(19) Europäisches Patentamt
European Patent Office
Office européen des brevets

(11) EP 1 386 965 A1

(12) **EUROPEAN PATENT APPLICATION**
published in accordance with Art. 158(3) EPC

(43) Date of publication:
**04.02.2004 Bulletin 2004/06**

(21) Application number: **02778901.5**

(22) Date of filing: **10.05.2002**

(51) Int Cl.7: **C12N 15/09**, C07K 14/705, C07K 16/28, C12N 5/10, C12Q 1/02, A61K 45/00, A01K 67/027

(86) International application number:
**PCT/JP2002/004570**

(87) International publication number:
**WO 2002/101035 (19.12.2002 Gazette 2002/51)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.05.2001 JP 2001140690**

(71) Applicant: **MOCHIDA PHARMACEUTICAL CO., LTD.**
**Shinjuku-ku Tokyo 160-8515 (JP)**

(72) Inventors:
- **NAKAMURA, Yusuke**
  **Yokohama-shi, Kanagawa 225-0011 (JP)**
- **SUGANO, Sumio**
  **Suginami-ku, Tokyo 167-0052 (JP)**
- **YAMASAKI, Fumiaki,**
  **MOCHIDA PHARMACEUTICAL CO. LTD.**
  **Tokyo 160-8515 (JP)**
- **FURUSAKO, Shoji,**
  **c/o MOCHIDA PHARM. CO. LTD.**
  **Tokyo 160-8515 (JP)**
- **OKAWA, Kazufumi,**
  **c/o MOCHIDA PHARM. CO. LTD.**
  **Tokyo 160-8515 (JP)**
- **TAKAHASHI, Tomohiro,**
  **c/o MOCHIDA PHARM. CO. LTD.**
  **Tokyo 160-8515 (JP)**

(74) Representative: **Wablat, Wolfgang, Dr.Dr.**
**Patentanwalt,**
**Potsdamer Chaussee 48**
**14129 Berlin (DE)**

# (54) NOVEL POTENTIAL-DEPENDENT CATION CHANNEL PROTEIN

(57) A gene and a protein participating in diseases of central nervous system and a method of efficiently evaluating an activity controller for the protein are provided.

A DNA comprising the nucleotide sequence represented by SEQ ID NO:1; a voltage-dependent cation channel protein encoded by the DNA; an antisense nucleic acid against the DNA sequence; a method of screening an agent capable of controlling a channel function of the protein with the use of the protein; an antisense nucleic acid against the DNA; and an antibody specific to the protein are provided.

EP 1 386 965 A1

## Description

### FIELD OF THE INVENTION

The present invention relates to a novel voltage-dependentcation channel protein, a DNA encoding said protein, a recombinant vector, a transformant produced by transformation with said vector, a specific antibody against said protein, an antisense nucleic acid inhibiting the expression of said protein, a method of screening a diagnostic agent, an inhibitor, a medicine and the like using said protein or said DNA encoding said protein, and a recombinant non-human animal.

### BACKGROUND OF THE INVENTION

Cells express various membrane proteins on cell membranes, nuclear membranes and any other biomembranes. These membrane proteins react with many biologically active substances very specifically and give their information to cells so that they control functions of cells or an entire organism.

As one of membrane proteins controlling cell functions, channel proteins, each protein forming a passage specific for a certain substance as pores penetrating a membrane and transporting the substance through the pores of the membrane, have been known. Each channel protein controls the transport of a certain substance by opening or closing the pores. This opening and closing portion is called as "a gate". The channel proteins are classified into "a ligand-gated channel" which is opened or closed in response to the presence of a biologically active substance or "a voltage-dependent channel" which is opened or closed depending on a membrane potential, based on mechanism how to open or close a gate.

As channel proteins, Na channel, K channel, Ca channel and the like in axon, postsynaptic membrane and excitable membrane participating in the transport of cations such as sodium($Na^+$), potassium ($K^+$), calcium ($Ca^{++}$) and the like; proton ($H^+$) channel (called as "F0") participating in the production of ATP in mitochondrial inner membrane; and the like have been known. These channel proteins are under investigation at levels of gene, protein and biomembrane, respectively.

The voltage-dependent cation channels such as Na channel, K channel, Ca channel and the like participate in the production of action potential and postsynaptic potential in an excitable cell membrane. It is estimated that these channels play an important role in the conduction of excitation between neurons. It is reported that many subtype proteins are present in each of the channel proteins. There is a conserved amino acid referred to as a specific ion selective filter every cation channel. In addition to the ion selective filter, K, Na and Ca channels are recognized to have a common characteristic that they have a similar primary structure. That is, K channel is a 6-times transmembrane type and Na and Ca channels are 24-times transmembrane type.

Many reports about voltage-dependent cation channel proteins and their genes are directed to those from brain, nervous tissues and the like. The voltage-dependent cation channel proteins and their genes are thought to play an important function in signal transmission in or between neurons. Especially, from the fact that many subtypes are present in each of the channel proteins, it is estimated that each subtype has unique physiological function. Since many cations including Na, K and Ca play very important biological function in vivo, to isolate the cation channel protein and specify its function is important scientificly and in the development of medicines in which the above function is applied. And, by using a voltage-dependent cation channel protein or a gene encoding this protein, efficient searching and evaluation of pharmaceutical candidate substances, especially specific channel function controller becomes possible.

Under the above circumstances, it is requested to find out a novel voltage-dependent cation channel protein from human and a gene encoding said protein. Further, the development of a method of efficiently screening an agent producing said protein and controlling an activity of said protein; an activity controller; a specific antibody; and an antisense nucleic acid are also needed.

An object of the present invention is to provide a novel voltage-dependent cation channel protein and a DNA encoding said protein. An additional object of the present invention is to provide a method of screening a channel function controller for said protein.

### SUMMARY OF THE INVENTION

The present invention relates to a novel gene isolated from human coronary arterial endothelial cells (hereinafter referred to "cae9577") and a voltage-dependent cation channel protein encoded by said gene(hereinafter referred to "CAE9577"). And, the present invention also provides a host cell transformed with said gene, a specific antibody against said protein, an antisense nucleic acid controlling the expression of said protein, a method of identifying a specific cation for CAE9577, a method of screening a channel function controller and a gene transferred non-human animal.

<nucleic acid>

The present invention provides a cae9577 gene encoding CAE9577 as described below in more detail. Specifically, the cae9577 gene means a DNA encoding a voltage-dependent cation channel protein comprising the amino acid sequence represented by SEQ ID NO:2. It includes a cDNA of SEQ ID NO:1 and 3 as well as a genomic DNA that said cDNA comes from. Although this gene can be isolated and identified from coronary arterial endothelial cells, the gene may be a DNA obtained by cloning using a genetic engineering technique such as a hybridization or a chemical synthetic technique such as a phosphoramidite method based on the sequence as disclosed herein. The form of the gene may be a cDNA, a genomic DNA and a chemically synthesized DNA, however not limited thereto.

The DNA of the present invention may be a single strand DNA. Alternatively, it may bind to a DNA or an RNA having the sequence complementary thereto to form a double- or triple-strand. The DNA may be labeled with an enzyme such as horseradish peroxidase (HRPO); a radioactive isotope; a fluorescent substance; a chemiluminescent substance; and the like.

If the nucleotide sequence of cae9577 is provided, a sequence of an RNA and a sequence of a complementary DNA and RNA are univocally determined. Therefore, it should be understood that the present invention also provides an RNA corresponding to the DNA of the present invention as well as a DNA and an RNA having a sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 1 or 3 under stringent conditions.

Variations of the nucleotide sequence represented by SEQ ID NO:1 or 3 are acceptable as long as they are hybridizable with the DNA comprising the nucleotide sequence represented by SEQ ID NO:1 or 3 under stringent conditions and a protein encoded by said DNA is a voltage-dependent cation channel protein. It should be understood that a DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of the DNA fragment by cleaving with a restriction enzyme are included within the present invention as long as the DNA mutant is hybridizable with the DNA represented by SEQ ID No. 1 or 3 under stringent conditions and encodes a voltage-dependent cation channel protein even if its sequence is different from the DNA sequence represented by SEQ ID No. 1 or 3.

The DNA mutant is acceptable as long as it has a homology with the DNA sequence represented by SEQ ID No. 1 or 3 of at least 70%, preferably at least 80%, more preferably at least 90%. The homology in DNA sequence can be analyzed by BLAST (J. Mol. Evol., Vol. 36, pp. 290-300 (1993); J. Mol. Biol., Vol. 215, pp. 403-410 (1990)). The term "hybridizable" means that a DNA is hybridizable with the nucleic acid represented by SEQ ID No. 1 or 3 by southern hybridization under stringent conditions. For example, if a probe labeled with DIG DNA Labeling kit (Cat No. 1175033 of Roche Diagnostics) is used, the hybridization is conducted in a DIG Easy Hyb solution (Cat No. 1603558 of Roche Diagnostics) at the temperature of, for example, 32°C (preferably 37°C, more preferably 42°C) and the membrane is washed in, for example, a 0.5 x SSC solution (containing 0.1% (w/w) SDS) at 50°C (preferably 65°C) (note: 1 x SSC is 0.15M NaCl and 0.015M sodium citrate).

The DNA comprising the nucleotide sequence represented by SEQ ID NO:1 or 3 or its partial fragment is believed to be useful as a specific probe for diseases in which the protein of the present invention participates such as psychopathic and neuropathic diseases.

The DNA of the present invention can be used to commercially produce CAE9577. And, the DNA can be used for testing the expression status of the protein of the present invention in a tissue by labeling with an enzyme or the like. That is, an expression amount of mRNA as an index of an expression amount of the protein of the present invention in a cell is confirmed by using the DNA as a probe so that a cell and culturing conditions of the cell suitable for the preparation of the protein of the present invention can be determined. In addition, diseases in which the protein of the present invention participates can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism) method, sequencing method and the like, using a part of the DNA of the present invention as a primer.

And, the DNA of the present invention can be used in gene therapy for diseases where the expression or activity of the protein of the present invention is impaired, by introducing the DNA of the present invention into *in vivo* cells.

The DNA of the present invention is very useful in the preparation of a transformant, the production of a recombinant protein CAE9577 using said transformant and the screening of a compound specifically inhibiting the expression of CAE9577.

The transformant of the present invention can be produced according to a method known for those skilled in the art. For example, the DNA of the present invention can be incorporated into a suitable host cell using any one of vectors commercially available or easily obtained by those skilled in the art. Then, the expression of the cae9577 gene within the host cell can be suitably controlled by placing the cae9577 gene under the influence of an expression control gene, typical examples of which are a promoter and an enhancer. This technique is suitable for being used in the production of the protein CAE9577 using the transformed host cell as well as the investigation of mechanisms how to regulate the expression of the cae9577 gene and the screening of an agent capable of controling the expression of said gene.

For example, by contacting any test substances with a cell transformed with the vector including the cae9577 gene under suitable conditions, an agent capable of enhancing or inhibiting the expression of the cae9577 gene can be searched among the test substances or evaluated.

By using the DNA of the present invention in combination with a known technique, a transgenic animal can be produced from a suitable animal such as mouse or the like. And, it is possible to produce the so-called knockout non-human animal in which a orthologue gene corresponding to the human cae9577 is destroyed in the animal by using the cae9577 gene of the present invention. By transducing the human cae9577 of the present invention into an animal whose endogenous gene is destroyed, a model animal having only human cae9577 can be produced. By observing the above transgenic animal, especially an animal expressing a large amount of the gene cae9577 or the protein CAE9577 of the present invention or an animal from which the gene cae9577 of the present invention is omitted, it is possible to identify a function of the gene cae9577 or the protein CAE9577. Further, this model animal is useful in development and evaluation of a drug targeting the human cae9577 transducted in said model.

It becomes possible to screen an agent specifically acting on the gene cae9577 or the protein CAE9577 or supplementing its function at *in vivo* level. The thus-screened agent is expected to be a drug as a biological controller in which CAE9577 specifically functions. Especially since a specific expression of the gene or the protein of the present invention is observed in brain, compounds obtained by screening the above transformant or transgenic animal are expected as effective therapeutic or preventive agents for psychopathic and neuropathic diseases caused by a dysfunction of brain.

<protein CAE9577>

A protein encoded by cae9577 is CAE9577 comprising the amino acid sequence represented by SEQ ID NO: 2. Since CAE9577 is homologous with Rb21 which is the voltage-dependent cation channel protein cloned from rat at a level of 98%, it is estimated to be a channel protein having a 24-times transmembrane structure similar to Rb21. This Rb21 from rat is confirmed to specifically express in brain, but it is estimated to have an unique ion selectivity since a conserved amino acid as a ion selective filter is EEKE, not EEEE in Ca channel or DEKA in Na channel (FEBS Letters, Vol. 445, pp. 231-236 (1999)). Generally, voltage-dependent Ca channel and Na channel each has a structure where 6-times transmembrane domain is repeated four times. A loop portion between S5 and S6 (sometime called as "pore loop") among 6 transmembrane segments (S1 to S6) contained in each domain (I to IV) functions as an ion selective filter. For example, in the loop portion of Ca channel, one glutamic acid per each domain is conserved, by which a selectivity for Ca ion is determined (the above-listed article; Proteins & Nucleic acid & Enzymes, Vol. 45, pp. 1013-1022 (2000)). By arranging a conserved amino acid in each domain in order from domain I to domain IV, the conserved amino acid is expressed with "EEKE". This is defined as a conserved amino acid as a ion selective filter. A conserved amino acid of CAE9577 as an ion selective filter was EEKE. Since it was confirmed that cae9577 specifically expresses in brain similarly to Rb21, CAE9577 is estimated to be a channel protein participating in the transport of a specific cation in central nervous system. The determination of a current of Rb21 from rat was tried in the article (FEBS Letters, Vol. 445, pp. 231-236 (1999)), an influx of either Na ion or Ba ion could not be determined so that an ion selectivity of Rb21 has not be elucidated. WO 01/83752 discloses IC23949 which is a protein homologous in amino acid sequence with the protein of the present invention at a level of 99%. IC23949 is estimated to be an ion channel protein from the characteristic of the amino acid sequence, but in practice the determination of an activity was not conducted and an ion selectivity of IC23949 was not clarified. On the other hand, the present inventors succeeded in the determination of an activity of CAE9577, thereby it becomes clear that Na ion can be flowed, but Ba ion and/or Ca ion cannot be flowed. Further, the present inventors found a characteristic property that a survival ratio is increased in oocytes in which CAE9577 expresses functionally, leading to the present invention. The above property has not been found in the prior Na channel protein.

Therefore, the elucidation of a mechanism how to transport a cation via CAE9577 can give a new aspect in a signal transduction in neurons and an agent of controlling a channel function acting on the channel protein is expected to be useful as a medicine based on a new action mechanism. Accordingly, it is expected that a screening of a channel function controller using CAE9577 is a very meaningful in the development of medicines.

A polypeptide or protein comprising an amino acid sequence wherein substitution, deletion and/or addition of one or more amino acids had occurred in the amino acid sequence represented by SEQ ID No. 2 are included within the scope of the present invention as long as it is a voltage-dependent cation channel protein.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln); aspartic acid (Asp) and asparagine (Asn); cysteine (Cys) and threonine (Thr); Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as nonpolar amino acids, they are understood to have similar properties to each other.

Non-charged polar amino acids include Ser, Thr, tyrosine (Tyr), Asn and Gln. Acidic amino acids include Asp and Glu. Basic amino acids include Lys, Arg and histidine (His). Even if the conservation as defined above is lost, many mutants maintaining functions essential for the protein (in the present invention, cation channel function) are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion and/or deletion of one or more amino acids in the amino acid sequence represented by SEQ ID No. 2 are included within the scope of the present invention as long as it is a voltage-dependent cation channel protein. The voltage-dependent cation channel protein as used herein means a protein having a function substantially homogeneous to that of CAE9577 which is the voltage-dependent cation channel protein comprising the amino acid sequence of SEQ ID NO:2, for example in a channel protein function for certain cation. Therefore, it should be understood that changes in molecular weight and the like of a protein caused by a variation of a strength of a channel function, a difference of a glycosylation binding and the like is acceptable as long as such a protein has a homogeneous cation specificity. Specifically, "a cation specificity" means an inflow of Na ion, preferably an inflow of Na ion without an inflow of Ba ion and/or Ca ion, more preferably additionally having a sensitivity for TTX (tetrodotoxin). As characteristic properties of CAE9577 which is the voltage-dependent cation channel protein of the present invention,

(i) a property of increasing a survival ratio of oocytes when the cae9577 gene is introduced into the oocytes as compared with a group in which the cae9577 gene is not introduced; and

(ii) a property of exhibiting a function when a mRNA from human neuroblastoma cell line SK-N-SH is co-introduced during the preparation of a transformant expressing the protein of the present invention;

can be mentioned. Having substantially homogeneous function to CAE9577 means that at least a cation specificity is homogeneous. Preferably, the properties (i) and/or (ii) are maintained.

The above changes in amino acids are found in the nature such as a mutation caused by a gene polymorphism or the like. Further, it can be produced artificially according to a known method for those skilled in the art, for example, mutagenesis using a mutagene such as NTG and site-specific mutagenesis using various recombinant gene techniques. The site and the number of the mutation of amino acids are not particularly limited as long as the resultant mutant protein is a voltage-dependent cation channel protein. The mutation number is generally within several tens of amino acids, preferably within 10 amino acids, more preferably within 1 or several amino acids.

Of course, the above protein may be in the form of a salt within the general knowledge of those skilled in the art. And, the protein of the present invention includes a single protein and proteins transformed into various forms including a fused protein with any other different protein. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins are proteins functions as a voltage-dependent cation channel protein.

The protein or its partial peptide of the present invention can be used in screening an agent capable of controlling an activity of said protein. The thus-screened compounds and the like are expected to be useful as effective therapeutic or preventive agents for diseases associated with the protein of the present invention.

<Method of screening specific cation and channel function modulator>

As mentioned above, a cation transported by RB21 and IC23949 which are highly homologous with CAE9577 are not identified. By specifying a specific cation transported by a channel protein, a complicated biological control mechanism will be elucidated so that a medicine having a novel action mechanism may be derived. Accordingly, identification, screening and evaluation of a specific cation for CAE9577 or an agent capable of controlling a channel function (modulator) are very significant.

The above cation can be determined by preparing a transformant using the gene cae9577, culturing the transformant expressing CAE9577 under a suitable condition and then confirming a cell response in the coexistence of a cation. A cell response considered is a change in a membrane potential, a membrane current and the like. Preferably, a mRNA from human neuroblastoma cell line SK-N-SH is co-introduced during the preparation of a transformant. The article of RB21 (FEBS Letters, Vol. 445, p. 231-236 (1999)) describes that in RB21, the determination of a current failed even by co-introducing a total RNA from brain. Thus, a system for determining an activity cannot be established by merely co-introducing an RNA from brain even if a specific expression is observed in brain. The present inventors especially noticed a human neuroblastoma cell line SK-N-SH and first found that the determination of a current in CAE9577 becomes possible by co-introducing a mRNA from SK-N-SH.

<Antibody>

Further, the present invention provides an antibody binding to CAE9577. The antibody of the present invention is an antibody specifically recognizing the entire protein or its partial peptide as an antigen. It includes a monoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as F $(ab')_2$ produced by digesting an immunoglobulin with, for example, pepsin, Fab produced by digesting an immunoglobulin with papain and the like, or a chimeric antibody, a humanized antibody and a human antibody. An antibody not only specifically recognizing CAE9577 or its partial peptide but also having the function of controlling an activity of CAE9577 is also included within the present invention. These antibodies are useful in investigation or clinical detection of CAE9577, clinical therapy of diseases which may be caused by CAE9577 and the like.

<Antisense nucleic acid>

The present invention provides the so-called antisense nucleic acid capable of inhibiting the biosynthesis of CAE9577 at a nucleic acid level *in vivo*. The antisense nucleic acid means a nucleic acid which binds to DNA or RNA involved in carrying a genetic information during either of a transcription stage from a genome region to a pre-mRNA essential for the production of mRNA encoding the CAE9577 protein, a processing stage from the pre-mRNA to a mature mRNA, a stage of passing through a nuclear membrane or a translation stage into a protein so as to affect the normal stream of the transmission of the genetic information and thereby to inhibit the expression of the protein. It may comprises a sequence complementary to the entire nucleotide sequence of the cae9577 gene or either part of the sequence. Preferably, it is a nucleic acid (including DNA and RNA) comprising a sequence corresponding to or complementary to the nucleotide sequence represented by SEQ ID NO: 1 or 3. When the mRNA transcripted from the genome region contains an intron structure or a untranslated region at 5' or 3'-terminal, an antisense nucleic acid corresponding to or complementary to the sequence of the non-translated region will have functions equivalent to those of the antisense nucleic acid of the present invention.

The antisense nucleic acid of the present invention includes a DNA and an RNA as well as all of derivatives similar to the DNA and the RNA in configuration and functions. The antisense nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives, in which at least one of a nuclease resistance, a tissue selectivity, a cell permeability and a binding power is improved. That is, the form of the nucleic acid is not limited as long as the nucleic acid can inhibit the activity and the expression of CAE9577.

And, the antisense nucleic acid having a nucleotide sequence complementary to a nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having a sequence complementary to that of these sites is also preferable since generally it can be expected to be very effective in inhibiting the expression.

In order to make the above antisense nucleic acid introduced into a cell and act efficiently, it is preferable that the length of the antisense nucleic acid of the present invention is 15 to 30 bases, preferably 15 to 25 bases, more preferably 18 to 22 bases.

The effect of the antisense nucleic acid of the present invention in inhibiting the expression can be evaluated by a known method, for example, by preparing an expression plasmid by linking a reporter gene such as luciferase and the like to the DNA containing a part of an expression control region, a 5'-non-translated region, a region near a translational initiation site or a translation region of the gene of the present invention, adding a test substance in a system such as a system comprising *in vitro* transcription (Ribo max systems; Promega) combined with *in vitro* translation (Rabbit Reticulocyte Lysate Systems; Promega) under the condition where the gene of the present invention is transcripted or translated and then determining an expression amount of the reporter gene.

The antisense nucleic acid of the present invention is useful as an agent for preventing or treating diseased associated with CAE9577 since it can inhibit the expression of CAE9577 in *vivo*.

DETAILED DESCRIPTION OF THE INVENTION

Embodiments of the present invention will be described below. As to principles and uses of a genetic engineering technique, a biochemical technique using proteins, cells, or animals as well as various devices such as a chromatography and a peptide synthesizer, their applications are not limited otherwise specified. Since techniques available by those skilled in the art, their principle and their use can be applied to the present invention, it should be understood that they are not limited by the following description.

<Nucleic acid>

Example of the method for obtaining the DNA of the present invention from a DNA library includes a method comprising screening a suitable genomic DNA library or cDNA library according to a screening method such as a screening method via hybridization, an immunoscreening method using an antibody and the like, amplifying a clone having the desired DNA and cleaving the DNA with a restriction enzyme or the like. In the screening method via hybridization, the hybridization can be conducted for any cDNA library using the DNA having the nucleotide sequence represented by SEQ ID No. 1 or a part thereof labeled with $^{32}$p or the like as a probe according to a known method (see, for example, Maniatis, T. et al., Molecular Cloning, a Laboratory Manual, Cold Spring Harbor Laboratory, New York (1982)). The antibody used in the immunoscreening method may be the antibody of the present invention as described below. The novel DNA of the present invention may be also obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990)) using sense and antisense primers prepared based on the nucleotide sequence of SEQ ID NO:1 or 3, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library may be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

And, the DNA of the present invention can be prepared based on the sequence as disclosed herein by a chemical synthetic technique such as a phosphoramidite method and the like.

The recombinant vector including the DNA of the present invention may have any form such as a cyclic form or a linear form. The recombinant vector may have any other nucleotide sequence in addition to the entire or a part of the DNA of the present invention, if necessary. "A part" means, for example, a DNA encoding a partial peptide of the protein of the present invention. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational initiation codon or a translational stop codon to the DNA of the present invention using a suitable synthetic DNA adaptor, and to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host used. The vector may be a plasmid. Alternatively, various viruses may be used, non-limiting examples of which include bacteriophage, vacuro virus, retro virus, vaccinia virus and the like. Thus, various elements generally used in genetic engineering techniques may be added to the gene of the present invention and alternatively the gene of the present invention may be altered according to the general genetic engineering technique. It should be understood that elements and technique disclosed herein is not exclusive and elements and technique available by those skilled in the art are used without particular limitation.

The gene of the present invention can be expressed under the control of a promoter sequence inherent in said gene. Using the expression system, an agent promoting or inhibiting the transcription of the gene of the present invention can be efficiently searched. Any other suitable expression promoter can be used by linking it to the promoter sequence inherent in said gene upstream of the gene of the present invention or replacing it with the promoter sequence. In this case, the promoter may be suitably selected depending on a host or an object of expression. For example, if a host is E. coli, a T7 promoter, a lac promoter, a trp promoter, a λPL promoter or the like can be used. If a host is a yeast, a PH05 promoter, a GAP promoter, an ADH promoter or the like can be used. If a host is an animal cell, a promoter from SV 40, a retrovirus promoter, an elongation factor 1α promoter or the like can be used. These lists are not exclusive.

A method for introducing the DNA into a vector is known (see J. Sambrook et al., Molecular Cloning, a Laboratory Manual, 2nd ed., Cold Spring Harbor Laboratory, New York (1989)). That is, each of the DNA and the vector is digested with suitable restriction enzymes and the resultant fragments are ligated with a DNA ligase.

<cae9577 gene recombinant non-human animal>

The present invention provides a recombinant non-human animal of cae9577 gene. The recombinant non-human animal of cae9577 gene includes transgenic non-human animals and knockout non-human animals. The cae9577 gene recombinant non-human animal is characterized by that the expression of the protein of the present invention in terms of its degree, its time, its site and the like is controlled by artificially introducing the gene encoding the protein of the present invention in a chromosome of the animal. Non-limiting example of non-human animals includes cattle, sheep, goat, porcine, mouse, horse, chicken and the like. Among the non-human animals, non-human mammalian animals are preferable.

By using the cae9577 gene of the present invention, a transgenic non-human animal can be produced. The transgenic non-human animal can be produced according to a routine method conventionally used in the production of transgenic animals (see, for example, "Experimental Manual of Genesis, published by Kodansha Scientific Ltd., edited by Motoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA prepared such that the cae9577 gene can be expressed is directly poured into a pronucleic oosperm obtained from a normal C57Black/6 mouse. More specifically, a construct is prepared by introducing the cae9577 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated offspring and confirmed whether or not the cae9577 gene is introduced by PCR or southern blotting, thereby a transgenic mouse is obtained.

The so-called "knockout mouse" can be produced based on the nucleotide sequence of cae9577 (or a mouse homologous gene of cae9577). The term "knockout mouse" used herein means a mouse in which an endogenous gene encoding the protein of the present invention is knocked out (inactivated). The knockout mouse can be produced by, for example, a positive-negative selection method via homologous recombination (see, for example, US patent Nos. 5,464,764, 5,487,992 and 5,627,059; Proc. Natl. Acad. Sci. USA, Vol. 86, pp. 8932-8935 (1989); Nature, Vol. 342, pp. 435-438 (1989)). Such a knockout mouse is one embodiment of the present invention.

Recently, the production of clone animals by nuclear transplantation in medium or large animals became possible. In this connection, transgenic and knockout animals have been practically produced using this technique. That is, a somatic cell or a germinal cell is subjected to homologous recombination based on the nucleotide sequence of cae9577 (or a homologous gene of cae9577 in each animal) in the same way as that applied to ES cells and then a nucleus is obtained from the resultant cell and used to obtain a clone animal. This animal is a knockout animal in which cae9577 (or a homologous gene of cae9577 in each animal) is lost. Alternatively, the cae9577 gene (or a homologous gene of cae9577 in each animal) is introduced in any cell of any animal and then the resultant nucleus is used to obtain a clone animal, thereby a transgenic animal can be produced. Such a knockout non-human animal and a transgenic non-human animal are one embodiment of the present invention irrespective of its species.

<Protein>

The protein of the present invention can be prepared from various organs naturally expressing said protein. Alternatively, it can be chemically synthesized in a peptide synthesizer (for example, Peptide Synthesizer Model 433A; Applied Biosystems Japan) or it can be produced by a recombination method using a suitable host cell selected from prokaryotic cells and eukaryotic cells. However, a genetic engineering technique and a recombinant protein produced thereby are preferable in view of purity.

A host cell to be transformed with the recombinant vector described in the previous section is not limitative. Many cells such as lower cells available in genetic engineering techniques, typical examples of which are E. coli, B. subtilis and S.cerevisiae; and animal cells, typical examples of which are insect cell, COS7 cell, CHO cell and HeLa cell, can be used in the present invention.

The transformant of the present invention can be obtained by transforming a suitable host cell with the recombinant vector of the present invention. As the method of introducing the recombinant vector described in the previous section into a host cell, some methods are known such as an electroporation, a protoplast method, an alkali metal method, a calcium phosphate precipitation method, a DEAE dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to obtain a culture mixture followed by purifying the protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, various affinity chromatographies including ion-exchange chromatography, hydrophobic chromatography and antibody chromatography, chromatofocusing, adsorption chromatography, reverse phase chromatography and the like. If necessary, HPLC systems or the like may be used to conduct several methods in a suitable order.

It is possible to express the protein of the present invention as a fusion protein with any other protein or tag such as glutathione S transferase, Protein A, hexahistidine tag, FLAG tag and the like. The thus-expressed fusion protein may be separated with a suitable protease such as thrombin, enterokinase and the like. This may be more effective for the preparation of the protein. For purifying the protein of the present invention, conventional methods may be suitably combined. Especially if the protein is expressed in the form of a fusion protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning, 2nd ed. (1989)).

As mentioned above, the protein of the present invention can be prepared in the form of a single protein or a fusion protein with any other different protein. The form of the protein of the present invention is not limited to them. Further, it is possible to transform the protein of the present invention to various forms. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins are proteins functions as voltage-dependent cation channel proteins.

The protein of the present invention can be used as an antigen for the preparation of an antibody and in screening an agent capable of binding to said protein or controlling an activity of said protein and therefore, it is useful.

When CAE9577 is present in a periplasm or a cytoplasm of a transformant, the transformant suspended in a suitable buffer is subjected to a treatment such as an ultrasonic treatment, a freeze-thawing treatment, a treatment with lysozyme or the like to destroy a cell wall and/or a cell membrane and further subjected to centrifugation, filtration or the like to obtain a membrane fraction containing the protein of the present invention. Next, this membrane fraction is solubilized with a suitable surfactant to prepare a crude solution, from which the desired protein can be isolated and purified according to the routine method.

<Antibody>

The antibody of the present invention may be polyclonal or monoclonal. Either antibody can be obtained by referring to a known method (see, for example, "Experimental Procedures in Immunology", edited by Japan Society for Immunology, published by Japan Society for Immunology), as describe below in brief.

For obtaining the novel antibody, an animal is inoculated with the protein of the present invention as an immunizing antigen and if necessary a suitable adjuvant such as Freund's complete adjuvant (FCA), Freund's incomplete adjuvant (FIA) and the like. If necessary, a booster at an interval of 2 to 4 weeks may be conducted. After the booster, an anti-serum is obtained by taking a blood sample. The protein of the present invention used as an antigen is that obtained in any method as long as it has a purity sufficient to be usable in the preparation of an antibody. A partial polypeptide of the protein of the present invention may be suitably used as an immunizing antigen. If the polypeptide used as an immunizing antigen is a low-molecular weight polypeptide, i.e. a polypeptide comprising about 10 to 20 amino acids, it may be linked to a carrier such as keyhole limpet hemocyanin (KLH) and the like and used as an antigen. Animals to be immunized include those conventionally used in immunological experiments by those skilled in the art such as rat, mouse, rabbit, sheep, horse, chicken, goat, pig, cattle and the like, among which preferably a species capable of producing the desired antibody is selected and used.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An antibody-producing cell such as a splenic cell, a lymphocyte and the like is taken from an immunized animal. The cell is fused with a myeloma cell line or the like according to a known method using polyethylene glycol, Sendai virus, an electric plus or the like to produce a hybridoma. Thereafter, a clone producing an antibody which binds to the protein of the present invention is selected and cultured. By purifying a supernatant of the culture of the selected clone, a monoclonal antibody is obtained. The purification may be conducted by suitably combining known methods such as salting-out, ion exchange chromatography, affinity chromatography and the like.

And, the novel antibody is also obtained by a genetic engineering technique. For example, a mRNA is obtained from a splenic cell or a lymphocyte of an animal immunized with the protein of the present invention or its partial polypeptide or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The desired antibody can be purified from the culture mixture by combined known methods. When the antibody is used for therapy, a humanized antibody is preferable with respect to immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using hypervariable regions of the monoclonal antibody (Method in Enzymology, Vol. 203, pp. 99-121 (1999)).

<Antisense nucleic acid>

The antisense nucleic acid can be prepared according to a known method (see, for example, edited by Stanley T. Crooke and Bernald Lebleu, in Antisense Research and Applications, published by CRC Publisher, Florida (1993)). If DNA and RNA are native, the antisense nucleic acid of the present invention can be obtained by synthesizing in a chemical synthesizer or conducting PCR using CAE9577 as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid can be obtained.

When the DNA and the antisense nucleic acid of the present invention is used as a diagnostic probe, they are labeled with a radioisotope, an enzyme, a fluorescent substance, a luminescent substance or the like according to a known method. Subsequently, a DNA or a mRNA is prepared from a specimen according to a known method and it is used as a test substance. This test substance is reacted with the labeled probe and then the reaction is washed to remove the labeled probe unreacted. If the test substance contains the cae9577 gene or RNA, said antisense nucleic acid binds thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or incorporating in a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, a stabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and the severity of the disease. Thus, it may be administered in an amount suitable to improve pathological conditions by the suitable method selected from oral, inhalation, transdermal, eye drop, intravaginal, intraarticular, intrarectal, intravenous, local, intramuscular, subcutaneous and intraperitoneal administrations.

<Screening method>

The present invention relates to a method of screening a cation specifically transported by the protein of the present invention or an agent capable of controlling the expression or a channel function of the protein of the present invention, which comprises using the protein of the present invention, a transformant expressing said protein, the DNA of the present invention, a recombinant vector including said DNA, a transformant produced by transformation with said vector, or a cae9577 gene recombinant non-human mammalian animal of the present invention.

More specifically, the screening method includes:

(1) a method of determining a change in current or potential in the coexistence of CAE9577 and a cation;
(2) a method of determining an influence of a test substance on a channel function of CAE9577 by contacting this protein with the test substance in the presence of a cation; and
(3) a method of screening an agent capable of controlling the expression of the protein of the present invention by comparing an expression level of the protein or the gene of the present invention in the presence /absence of a test substance; and the like. Example of the methods (1) and (2) are an assay using a biomembrane or cells expressing CAE9577 in Xenopus oocyes or a mammalian animal cell system. A channel function to be determined includes a change in current or potential. This can be determined according to a known method (in case of Xenopus oocyes, see Proc. Natl. Acd. Sci. USA, Vol. 89, pp. 554-558(1992); in case of mammalian animal cells, see Am. J. Physiol., Vol. 274, pp. H1643-H1654(1998)). More specifically, a method as illustrated in Example 5 can be exemplified. Example of the method (3) is a method comprising preparing an expression plasmid by linking a reporter gene such as luciferase or the like to the DNA containing a part of an expression control region, a 5'-non-translated region, a region near a translational initiation site or a translation region of the cae9577 gene and determining an expression amount of the reporter gene under the condition where the gene of the present invention is transcripted or translated in the presence/absence of a test substance so as to confirm a transcriptional promotion activity or a transcriptional inhibitory activity of the test substance. The screening method of the present invention comprises the steps of contacting a test substance with the protein of the present invention, a transformant expressing said protein, the DNA of the present invention, a recombinant vector including said DNA, a transformant produced by transformation with said recombinant vector or a cae9577 gene transferred non-human animal; detecting a difference in an activity of the protein of the present invention or an expression level of the DNA of the present invention between a group with the addition of the test substance and a group without the addition of the test substance; and selecting the test substance showing the difference as an agent capable of controlling an activity of the protein of the present invention or an agent capable of controlling the expression of the DNA of the present invention. Preferably, a m-RNA from human neuroblast cell line SK-N-SH is co-transfected during the preparation of the transformant expressing the protein of the present invention.

An agent capable of controlling an activity of the protein of the present invention may be an agent capable of either enhancing(agonist) or inhibiting (antagonist) the activity of the CAE9577 protein. An agent capable of controlling the expression of the DNA of the present invention may be an agent capable of either promoting or inhibiting the expression of the cae9577 gene. An agent capable of inhibiting the expression is preferable. For confirming whether a test substance controls an activity of the protein of the present invention or controls the expression of the DNA of the present invention, a difference in the activity of the protein or the expression level of the DNA is determined between the addition and no addition of a test substance in a system capable of confirming the activity of the protein or a system capable of confirming the expression of the DNA. The expression level of the DNA may be determined on the basis of an expression strength of the cae9577 gene into mRNA or the protein. Instead of the expression level of the cae9577 gene or the CAE9577 protein per se, an expression level of a reporter gene may be detected. The reporter-assay system means an assay system in which an expression amount of a reporter gene arranged understream of an expression control region is determined so as to screen an agent affecting the expression control region. Examples of the expression control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The expression control region and the 5'-non-translated region of the gene of the present invention can be obtained according to a known method (see "New Experimental Protocol in Cell Engineering", published by Shojunsha Co., Ltd.(1993)). Having function of inhibiting (or suppressing) or enhancing (or promoting) means that a determined value as to the activity of the protein or the expression level of the DNA is different between a group with the addition of a test substance and a group without the addition of a test substance. For example, the inhibition (or suppression) or the enhancement (or promotion) ratio calculated by the following equation is 10% or higher, preferably 30% or higher, more preferably 50% or higher, even preferably 70% or higher, especially preferably

90% or higher.

inhibition (or suppression) or enhancement (or promotion) ratio

(%) =

[absolute value of (determined value of a group without the addition of a test substance) minus (determined value of a group

with the addition of a test substance)] / (determined value of

a group without the addition of a test substance) * 100

Either inhibition or enhancement is suitably determined depending on the kind of a system capable of confirming an activity of the protein or a system capable of confirming the expression of the DNA. For example, if a system capable of confirming an activity of the protein is a system for determining a current as shown in Example 5, a current value can be determined. When the determined value in a group with the addition of a test substance is lower than that in a group without the addition of a test substance, the test substance can be judged to have an action of inhibiting the activity of the CAE9577 protein. Of course, if values from background and/or noises is contained in a determination system, they should be subtracted.

Since the protein of the present invention is a voltage-dependent cation channel protein specifically expressing on a brain, compounds obtained through the search using the screening method or transgenic animal described above are expected to be effective therapeutic or preventive agents for diseases of central nervous system. Non-limiting examples of a test substance include proteins, peptides, oligonucleotides, synthetic compounds, naturally occurring compounds, fermented products, cell extracts, plant extracts, animal tissue extracts and the like. The test substance may be either new or known.

## BRIEF EXPLANATION OF DRAWINGS

Fig. 1 shows an expression distribution of the cae9577 gene in vivo. The vertical axis represents a relative expression amount.

Fig. 2 shows a genome structure of the cae9577 gene.

Fig. 3 shows the results of electrophysiological studies in CAE9577 expressed oocytes as shown in Example 5.

Fig. 3-1 shows the results of the measurement of Na current in (1) 20 ng/oocyte of CAE9577 mRNA. CONT indicates the absence of TTX.

Fig. 3-2 shows the results of the measurement of Na current in (2) each 10 ng/oocyte of CAE9577 mRNA + mRNA from SK-N-SH cell. CONT indicates the absence of TTX.

Fig. 3-3 shows the results of the measurement of Na current in (3) 20 ng/oocyte of mRNA from SK-N-SH cell. CONT indicates the absence of TTX.

Fig. 3-4 shows the results of the measurement of Ca current in (1) 20 ng/oocyte of CAE9577 mRNA. CONT indicates the use of a Ba buffer in an extracellular fluid and Ba FREE indicates the use of a Ba/Ca free buffer in an extracellular fluid.

Fig. 3-5 shows the results of the measurement of Ca current in (2) each 10 ng/oocyte of CAE9577 mRNA + mRNA from SK-N-SH cell. CONT indicates the use of a Ba buffer in an extracellular fluid and Ba FREE indicates the use of a Ba/Ca free buffer in an extracellular fluid.

Fig. 3-6 shows the results of the measurement of Ca current in (3) 20 ng/oocyte of mRNA from SK-N-SH cell. CONT indicates the case of using a Ba buffer in an extracellular fluid and Ba FREE indicates the case of using a Ba/Ca free buffer in an extracellular fluid.

## EXAMPLES

The present invention will be described in more detail by referring to the following examples which are not to be construed as limiting the scope of the invention.

Example 1 : Cloning of a gene, cae9577

(1) Construction of full length cDNA library according to oligocapping method

A poly(A)$^+$RNA was prepared from human coronary arterial epithelial cells according to the method of Sambrook et al. (Molecular Cloning. A Laboratory Manual, 2nd edition, Cold Spring Harbor Laboratory, Cold Spring Harbor, NY) using an oligo(dT)-cellulose. Next, 5 to 10 μg of the poly(A)$^+$RNA was reacted with 1. 2U of Bacterial Alkaline Phosphatase (hereinafter abbreviated as "BAP") (TaKaRa) in a buffer containing 100mM of Tris-HCl (pH 8.0), 5mM of 2-mercaptoethanol and 100U of RNasin (Promega) at 37°C for 40 minutes so as to dephosphorylate the poly(A)$^+$RNA having no cap structure. Thereafter, the reaction liquid was extracted with a mixture of phenol and chloroform (1:1) twice and the poly(A)$^+$RNA was collected as ethanol precipitates. The thus-collected poly(A)$^+$RNA was treated with 20U of Tobacco acid pyrophosphatase (hereinafter abbreviated as "TAP") (Maruyama and Sugano, Gene, Vol. 138, pp. 171-174) in a buffer containing 50mM of sodium acetate (pH 5.5), 1mM of EDTA, 5mM of 2-mercaptoethanol and 100U of RNasin at 37°C for 45 minutes so as to remove the cap structure. Thereafter, the reaction liquid was extracted with a mixture of phenol and chloroform (1:1) twice and subjected to ethanol precipitation to collect a BAP-TAP treated poly(A)$^+$RNA.

2 to 4 μg of the thus-collected poly (A) $^+$RNA was ligated to 0.4 μg of 5'-oligomer (5'-AGC AUC GAG UCG GCC UUG UUG GCC UAC UGG-3'). This reaction was conducted with 250U of RNA ligase (TaKaRa) in a buffer containing 50mM of Tris-HCl (pH 7.5), 5mM of $MgCl_2$, 5mM of 2-mercaptoethanol, 0.5mM of ATP, 25% of PEG8000 and 100U of RNAsin at 20°C for 3 to 16 hours. Thereafter, the oligomer unreacted was removed, and a cDNA was synthesized. That is, 2 to 4 μg of the oligocapped poly(A)$^+$RNA was mixed with 10pmol of a dT adaptor-primer (5'-GCG GCT GAA GAC GGC CTA TGT GGC CTT TTT TTT TTT TTT TTT-3') and the mixture was reacted with SuperScript II RNase H- Reverse Transcriptase (Gibco BRL) in a buffer attached thereto at 42°C for 1 hour.

The reaction was conducted with 15mM of NaOH at 65°C for one hour to remove the RNA as a template and then a cDNA was amplified. 1 μg of the cDNA synthesized from the oligocapped poly(A)$^+$RNA was mixed with 16 pmol of a sense primer 1 (5'-AGC ATC GAG TCG GCC TTG TTG-3') and an antisense primer 1 (5'-GCG GCT GAA GAC GGC CTA TGT-3') and amplified using XL PCR kit (Perkin-Elmer). The reaction condition for PCR was 5 to 10 cycles, each cycle comprising heating at 94°C for 1 minute, at 58°C for 1 minute and at 72°C for 10 minutes. The PCR product was extracted with a mixture of phenol and chloroform (1:1) and collected as ethanol precipitates. Thereafter, the thus-collected product was digested with SfiI and subjected to electrophoresis on agarose gel to separate a cDNA of 1, 000 bp or higher. This cDNA was inserted into DraIII site of pME18S-FL3 (GenBank accession No. AB009864) which was an expression vector for mammalian cells. Since this DraIII site of pME18S-FL3 was asymmetrical, the terminal of the cDNA fragment was a SfiI site complementary thereto and therefore the cDNA fragment was inserted unidirectionally.

(2) Sequencing of cDNA clone and analysis of information about its deduced protein

A plasmid was prepared from the cDNA library constructed by the method described in the above section (1) by means of a pI-100 robot (KURABO). Each clone was sequenced and the resultant sequence was used as data base. A sequence reaction was conducted using AutoCycle sequencing kit (Amersham Pharmacia) and R.O.B. DNA processor (Amersham Pharmacia) according to the provider's protocol, and sequencing was conducted in ALF DNA sequencer (Amersham Pharmacia).

The plasmid C-CAE09577 obtained by the method (1) contained a cDNA comprising the nucleotide sequence of 2471 base pairs represented by SEQ ID NO:6, containing an open reading frame comprising the nucleotide sequence of 903 base pairs represented by SEQ ID NO:4, encoding a novel protein comprising 301 amino acids represented by SEQ ID NO:5.

The amino acid sequence comprising 301 amino acids was searched to find a local agreement in sequence. For searching the homology, BLAST (Altschul SF., J. Mol. Evol., Vol. 36, pp. 290-300 (1993); Altschul SF., J. Mol. Biol., Vol. 215, pp. 403-10 (1990)) was used.

The deduced protein sequence was subjected to BLAST homology searching with Genbank (http://www.ncbi.nlm.nih.gov/) protein data base. The homology with rat putative four repeat ion channel (GenBank Accession: AAC68885) was found over 301 residues at a level of 95%. The homology with fruit-fly CG1517 gene product (GenBank Accession: AAF48365) was found over 170 residues at a level of 83%. The homology with nematode similar to voltage-gated calcium channel subunits (GenBank Accession : AAA81424) was found over 155 residues at a level of 75%.

Example 2 : _Analysis of expression profile by ATAC-PCR

Adaptor-tagged competitive PCR (ATAC-PCR) was conducted by contracting to the outer research institute according to the method described in Nucleic Acids Research, Vol. 25, pp. 4694-4696 (1977).

Human RNA used for analysis was as follows: A total RNA was prepared from human coronary arterial endothelial cells (HCAEC) and human placental tissue according to a routine method. While, a total RNA was prepared according to a routine method after culturing leukocytes from human peripheral blood in the presence of 50 μg/ml of PHA for 24 hours. And, a total RNA of each of lung, kidney, pancreas, small intestine, thymus, spleen, heart, uterus, testis, prostate and skeletal muscle was purchased from Clontech. A total RNA of each of liver, colon, leukocytes and brain was purchased from BioChain Institute. A single strand cDNA was synthesized from 6 μg of each total RNA according to a routine method using an oligo (dT) - primer. As a reverse transcriptase, SuperScript II RNase $H^-$Reverse Transcriptase (GibcoBRL) was used. A sequence of a cae9577 specific primer used in PCR was 5'-GGC CAT TGA CAT ATA AGG AA-3'. As the result, a specific and significant expression was found in brain (Fig. 1).

Example 3 : Cloning of full length cDNA encoding CAE9577

(1) Cloning of cDNA

The amino acid sequence encoded by C-CAE09577 showed a high homology with a sequence at C terminal in rat putative four repeat ion channel (GenBank Accession: AAC68885). And, the same reading frame was present upstream of Met which is perhaps a translation initiation codon and this 10 amino acids also showed a high homology with the amino acid sequence of AAC68885. Thus, it is anticipated that C-CAE09577 is a human homolog gene of rat putative four repeat ion channel provided that a partial cDNA sequence in which about 4kb from 5' terminal was deleted.

The method used for cloning C-CAE09577 shown in Example 1 is referred to as "an oligocapping method". According to this method, a full length cDNA is obtained in principle. However, an average length of cDNA prepared according to the oligocapping method is about 200 nucleotides (Experimental Medicine, Vol. 18, pp. 1575-1580 (2000)) and therefore, a full length cDNA is hardly obtained from a clone having longer ORF. In such a case, a method comprising designing a primer from a gene considered to be a homolog and then cloning is proposed. However, generally a nucleotide sequence is not completely conserved even between homologs of neighbor species by using this method and therefore it cannot be correctly cloned depending on the design of a primer.

Based on the cDNA sequence (GenBank Accession: AF078779) of rat putative four repeat ion channel, a human genome sequence was subjected to homology searching so that a sequence near to a translation initiation codon of CAE9577 was confirmed. Next, a sense primer (5'-CTG TGG TTT TGT GCT TGC TC-3') was designed at 38bp upstream of the initiation codon (ATG) and an antisense primer (5'-GCT TGC GGT ATC ATT TCT A-3') was designed at about 250 bp downstream of the stop codon. Then, PCR cloning was conducted using a cDNA prepared from human umbilical vein endothelial cells (HUVEC) as a template. The resultant fragment of about 5.5 kb was inserted in EcoRV site of pBluescript II Sk(+) (STRATAGENE) to confirm the sequence. As the result, this plasmid contained an open reading frame comprising 5214 nucleotides represented by SEQ ID NO: 1, encoding a novel protein comprising 1738 amino acids represented by SEQ ID NO: 2. Combined with C-CAE09577 sequence cloned in Example 1, the full length of the cDNA sequence of cae9577 was 6789 nucleotides represented by SEQ ID NO: 3.

Next, a local sequence agreement against the amino acid sequence comprising 1738 amino acids was searched. For searching the homology, BLAST (Altschul SF, J. Mol. Evol., Vol. 36, pp. 290-300 (1993)) was used.

The protein sequence was subjected to BLAST homology searching with Genbank protein data base (http://www.ncbi.nlm.nih.gov/). As the result, the homology with rat putative four repeat ion channel (GenBank Accession : AAC68885) was found over 1738 residues at a level of 98%. The homology with fruit-fly CG1517 gene product (GenBank Accession : AAF48365) was found over 1634 residues at a level of 71%. The homology with nematode similar to volatage-gated calcium channel subunits (GenBank Accession : AAC) was found over 1626 residues at a level of 65%.

Motifs are represented as consensus sequences of functional sites (for example, an active site such as an enzyme; a binding site such as a ligand or an effector; a modifying site such as a phosphorylation) identified by experiment and the like. Since especially important functional sites are often conserved even after evolution, they are used as an index characterizing a conserved sequence specifically appearing in a protein expressing certain function and also an analogous protein family. Thus, it is expected to lead an interpretation which directly relates to functions by searching a motif rather than a homology.

In PROSITE data base (http://www.expasy.ch/prosite/) which is a motif data base, a motif is represented by a pattern of a consensus sequence. Alternatively, it is represented by a profile in which a score based on an appearance frequency or the like is expressed with a matrix for each amino acid to each position within the motif.

As to the functions of the deduced protein sequence, patterns were searched for PROSITE data base. As the result, motifs where asparagines at Nos. 210, 216, 859, 1064, 1371 and 1449 are sugar chain binding site were found.

Using HMMER (R. Durbin, S. Eddy, A. Krogh, G. Mitchison, Cambridge University Press (1998)) which is a program for screening a homology using a hidden Markov model, pfam (http://www.sanger.ac.uk/Pfam/) of a protein domain database was subjected to screening. As the result, ion transport protein domains [Pfam Accession : PF00520] were found at amino acid Nos. 32 to 321, Nos. 380 to 598, Nos. 884 to 1155 and Nos. 1206 to 1446 as a significantly homologous domains.

Amino acids (280E, 554E, 1115K, 1989E) which are estimated to be important as an ion selective filter were conserved between rat putative four repeation channel (GenBank Accession : AAC68865). Thus, they were different from generally known Ca channel (EEEE) and Na channel (DEKA).

(2) Genome sequence of cae9577

Based on the sequence of SEQ ID NO:3 obtained in the above (1), a human genome sequence was again subjected to homology searching. As the result, it was confirmed that homology sequences were found in clones RP11-75C5 (Accession No. AL356778), RP11-4417 (Accession No. AL354891) and RP11-430M15 (Accession No. AL138707) of 13th chromosome. Further, an intron portion was confirmed based on GT-AG rule and the position of an exon was searched. As the result, it was clarified that cae9577 comprises 43 exons. As shown in Fig. 2, Nos. 1 to 6 exons were arranged in RP11-75C, Nos. 7 to 12 exons were arranged in RP11-4417, and Nos. 13 to 43 exons were arranged in RP11-430M15. When the exon sequence of SEQ ID NO: 3 and that of the genome were compared, the base C at No. 4454 of SEQ ID NO: 3 was replaced with A in the genome sequence. Although this base is the third codon of Ile at No. 1472 of SEQ ID NO: 2, A from the genome sequence also encodes Ile. An amino acid sequence deduced from cDNA was identical with an amino acid sequence deduced from the genome sequence. And, in 3'-non-translated region, one base was replaced at five positions, two bases were deleted at one position, and two bases were inserted in one position.

<u>Example 4 : electrophysiological properties in CAE9577 expressed mammalian cells</u>

(1) Construction of expression plasmid

A plasmid expressing CAE9577 in mammalian cells was constructed according to the following method. The plasmid obtained in Example 3 was digested with KpnI and EcoRI to collect a fragment of about 5.4 kb. Next, pcDNA3.1 (+) was similarly digested with KpnI and EcoRI, to which the above fragment was inserted. It was transformed with JM109 to obtain an expression plasmid pcDNA09577. This plasmid pcDNA09577 was deposited in International Patent Organism Depositary (IPDO) (Tsukuba City, Ibaragi Prefecture, Japan) of National Institute of Advanced Industrial Science and Technology as FERM BP-8034 on November 7, 2001.

(2) Expression of CAE9577 and tests of electrophsiological properties

The pcDNA09577 obtained in the above (1) is introduced in cells CHO-K1 from Chinese Hamster Ovary (ATCC No. CCL-61). That is, 50 µl of FuGENE6 (Roche Diagnostics) is mixed with 12.5 µg of the pcDNA09577 according to the provider's protocol and the mixture is added to CHO-K1 cells grown in a semiconfluent condition in a 150cm$^2$ flask. After the cells are cultured at 37°C in 5% $CO_2$ for 24 hours, they are taken off and inoculated on a cover glass. After the cells are cultured for additional 24 hours, the cover glass is put in a chamber fixed on a stage of an inverted microscope and a solution containing a suitable ion described below is perfused. A glass tube micropippet for patch clamping (patch electrode) is prepared by pulling a glass tube using a two-stage puller and then heat polishing its tip using a microforge. The patch electrode is filled with a solution comprising 130mM CsCl, 10mM NaCl, 0.4mM $MgCl_2$, 5mM Mg-ATP, 5mM glucose, 10mM HEPES, 5mM EGTA and a CsOH solution for adjusting a pH at 7.3. The patch electrode is applied to the cells to form a giga-seal. A current is recorded according to a whole cell recording method using a patch clamping amplifier. For recording the data, a commercially available software (pCLAMP7 of Axon Instruments) is used. The data are subjected to a filter of 5 kHz and recorded at a sampling frequency of 50 kHz. When Na current is recorded, an extracellular fluid to be perfused has a composition comprising 10mM CsCl, 130mM NaCl, 2mM $CaCl_2$, 1.2mM $MgCl_2$, 2mM $CoCl_2$, 11mM glucose, 20mM HEPES and a CsOH solution for adjusting a pH at 7.3. When Ca current is recorded, an extracellular fluid to be perfused has a composition comprising 10mM $BaCl_2$, 140mM TEA-Cl, 5mM CaCl, 11mM $MgCl_2$, 5mM glucose, 10mM HEPES and a TEA-OH solution for adjusting a pH at 7.3.

<u>Example 5 : Tests of electrophysiological properties in CAE9577 expressed oocytes</u>

(1) Extraction of mRNA from SK-N-SH cells

A polyA RNA was extracted from human neuroblastoma cell line SK-N-SH (ATCC Number: HTB-11) using Fast Track 2.0 (Invitrogen). Thereafter, the thus-extracted RNA was passed through a cellulose column and purified with phenol and chloroform to obtain a mRNA from SK-N-SH cells.

(2) Preparation of mRNA encoding CAE9577

The pcDNA9577 prepared in Example 4 was linearlized with BssHII and purified with phenol and chloroform. The linear DNA was subjected to in vitro transcription using T7 promoter as a template. Thereafter, the synthesized product was purified with phenol and chloroform, subjected to electrophoresis and determined for absorbance so as to confirm the purity of the synthesized RNA and its amount. While, in order to stabilize the synthesized RNA, poly(A) was added to 3'-terminal of the synthesized RNA using Poly(A)Tailing Kit (Ambion).

(3) Preparation of oocytes

Oocytes extracted from Xenopus (Shiki Ieda Kagaku) were isolated by removing follicles with collagenase. Fifth- or sixth-stage oocytes were selected in a ND buffer (96mM NaCl, 2.0mM KCl, 1.0mM $MgCl_2$, 1mM $CaCl_2$, 5mM HEPES, pH 7.5). The oocytes after the selection were incubated at 20°C overnight, to which the mRNA was microinjected. The type and the amount of the thus-injected mRNA were as follows:

(i) CAE9577 mRNA as prepared in Example 5-(2): 20 ng/oocyte
(ii) CAE9577 mRNA as prepared in Example 5-(2) + mRNA from SK-N-SH cells as prepared in Example 5-(1): each 10 ng/oocyte
(iii) mRNA from SK-N-SH cells as prepared in Example 5-(1): 20 ng/oocyte

The oocytes after the microinjection were incubated in a ND buffer at 20°C for 72 hours. And, after 24, 48 and 72 hours, the oocytes were tested for survival ratio.

(4) Current measurement in oocytes

After the incubation for 72 hours, a current measurement in the oocytes with a suitable condition was conducted according to the following method.

For the measurement of Na current, an extracellular fluid comprising a Na buffer (100mM NaOH, 100mM methanesulfonic acid, 2mM $CaCl_2$, 10mM HEPES, pH 7.3) was used. Tetrodotoxin (TTX of Wako Pure Chemical Co., Ltd.) was dissolved in the Na buffer and the solution was directly added dropwise to the oocytes. The holding potential was set at -100 mV and a depolarizing pulse of 20 ms was applied over -90 mV to +80 mV on every 10 mV.

For the measurement of Ca current, an extracellular fluid comprising a Ba buffer (50mM NaCl, 2mM KCl, 40mM $BaCl_2$, 5mM HEPES, pH 7.5) was used. For the measurement under the condition of the absence of Ba in an extracellular fluid, a Ba/ca free buffer (90mM NaCl, 2mM KCl, 5mM HEPES, pH 7.5) was used. The holding potential was set at -80 mV and a depolarization pulse of 200 ms was applied over -70 mV to +40 mV on every 10 mV.

(5) Results

<u>Survival ratio of oocytes</u>

As shown in the following table, the survival ratio of oocytes in which both CAE9577 mRNA and mRNA from SK-N-SH cells were simultaneously introduced was higher than that of oocytes in which either CAE9577 mRNA or mRNA from SK-N-SH cells was introduced. The results suggest that the CAE9577 mRNA product together with a protein contained in a product of mRNA from SK-N-SH cells form a functional protein so that it may enhance a function associated with a survival ratio of cells or inhibit any process leading to cell death. It is estimated that the above property is useful in the treatment of degenerative diseases of brain such as Parkinson's disease, considering that CAE9577 originates from brain.

| Viability of oocytes | | | | |
|---|---|---|---|---|
| introduced gene | number of expressed cells | survival ratio (%) | | |
| | | after 24 hours | after 48 hours | after 72 hours |
| (i) | 30 | 70.0 | 66.7 | 63.3 |
| (ii) | 46 | 95.7 | 89.1 | 80.4 |

(continued)

| Viability of oocytes | | | | |
|---|---|---|---|---|
| introduced gene | number of expressed cells | survival ratio (%) | | |
| | | after 24 hours | after 48 hours | after 72 hours |
| (iii) | 38 | 81.6 | 78.9 | 63.2 |
| (i) CAE9577 mRNA : 20 ng/ocyte<br>(ii) CAE9577 mRNA + mRNA from SK-N-SH cells : each 10 ng/oocyte<br>(iii) mRNA from SK-N-SH cells : 20 ng/oocyte | | | | |

Na current

In oocytes in which merely either CAE9577 mRNA or mRNA from SK-N-SH cells were expressed, an inward current perhaps due to a leak was produced in a depolarizing pulse and a channel-like current was not observed. In oocytes which CAE9577 mRNA and mRNA from SK-N-SH cells were co-expressed, an inward current in response of the stimulation of depolarization was produced. Its peak was observed near at -70 mV. And, this current was partially inhibited by TTX (Figs. 3-1 to 3-3).

Ba Current

A signal showing the change in current was not obtained from either cells. The known Ca channel-like activity was not observed (Figs. 3-4 to 3-6).

In summary, it can be believed that a product of CAE9577 mRNA together with a protein contained in a product of mRNA from SK-N-SH cells form a channel through which Na ion is flowed under the conditions as used in Example.

EFFECT OF THE INVENTION

CAE9577 of the present invention is useful for the identification of a cation specifically transported by said CAE9577. And, it is useful for the development of an agent capable of controlling a cation transporting mechanism so as to effect a new control mechanism in vivo.

## Claims

1. The following DNA (a) or (b):

   (a) a DNA comprising the nucleotide sequence represented by SEQ ID NO:1, or
   (b) a DNA hybridizable with the DNA of SEQ ID NO:1 under stringent conditions and encoding a voltage-dependent cation channel protein.

2. A protein encoded by the DNA as defined in claim 1.

3. The following protein (a) or (b):

   (a) a protein comprising the amino acid sequence represented by SEQ ID NO:2, or
   (b) a voltage-dependent cation channel protein comprising an amino acid sequence wherein deletion, substitution or addition of one or more amino acids has occurred in the amino acid sequence of SEQ ID NO:2.

4. A protein as defined in claim 3 wherein a conserved amino acid as an ion selective filter is EEKE.

5. A protein as defined in claim 3 which has an activity of flowing Na ion.

6. A DNA encoding the protein as defined in any one of claims 3 to 5.

7. A recombinant vector including the DNA as defined in claim 1 or 6.

8. A transformant produced by transformation with the recombinant vector as defined in claim 7.

9. An antisense nucleic acid inhibiting the expression of the protein as defined in claim 2 or 3.

10. An antisense nucleic acid as defined in claim 6 whose nucleotide sequence is a sequence complementary to the entire or a part of the DNA as defined in claim 1 or the nucleic acid represented by SEQ ID No. 3.

11. An antibody against the protein as defined in claim 2 or 3 or its partial peptide.

12. A method of determining a cation specifically flowing through the protein as defined in claim 2 or 3, which comprises contacting the protein or the transformant expressing the protein with the cation and then determining a response on the change in current or potential.

13. A method of screening an agent capable of controlling a channel function of the protein as defined in claim 2 or 3, which comprises contacting the protein or the transformant expressing the protein with a test substance and then determining the change in channel function of the protein.

14. A method as claimed in claim 13 wherein a mRNA from human neuroblastoma cell line SK-N-SH is co-introduced during the preparation of a transformant expressing the protein as defined in claim 2 or 3.

15. A method of screening an agent capable of inhibiting the expression of the DNA as defined in claim 1, which comprises contacting the vector as defined in claim 7 or the transformant as defined in claim 8 with a test substance and then detecting the change in expression level of the DNA as defined in claim 1.

16. A recombinant non-human animal of cae9577 gene.

Fig. 1

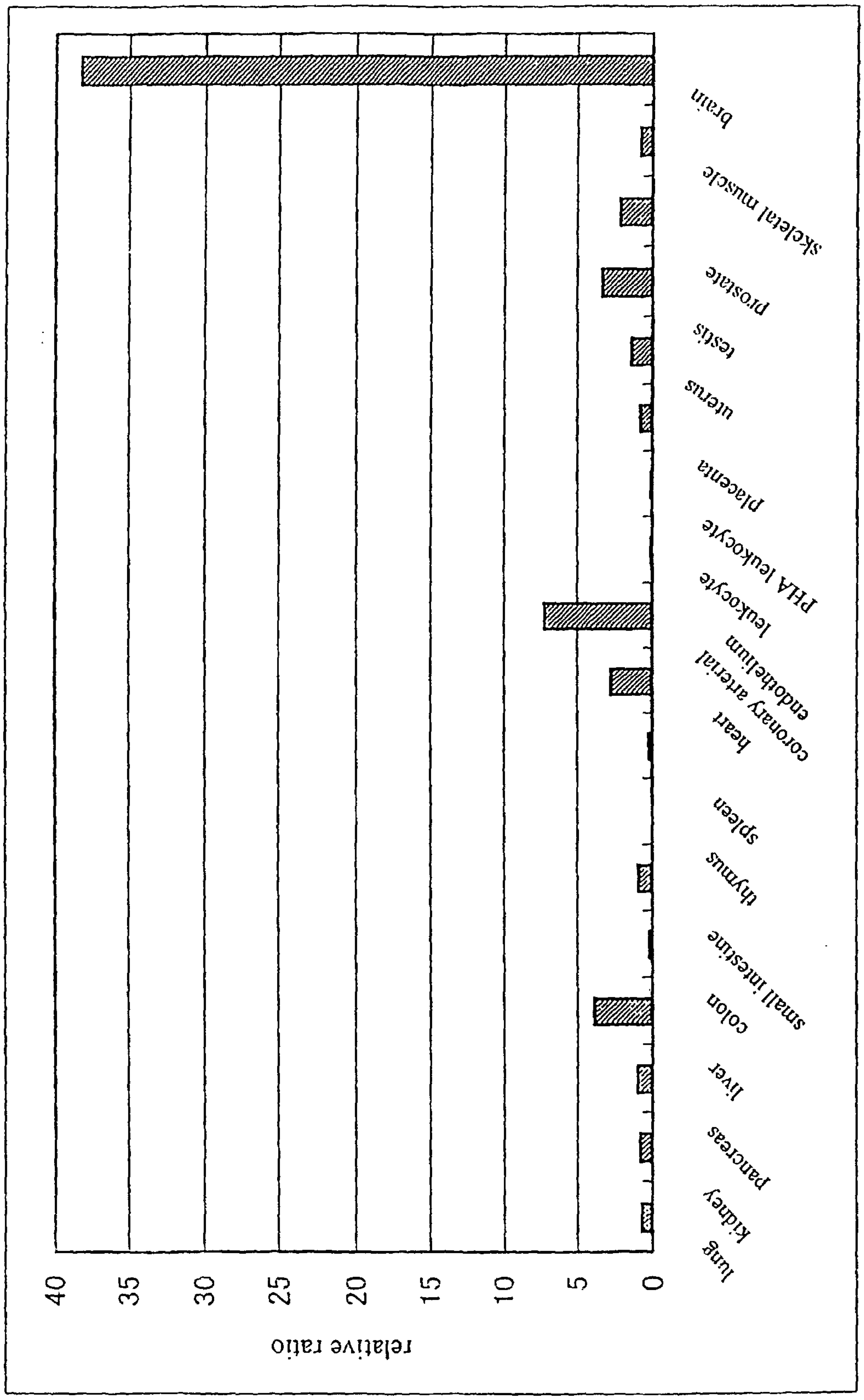
relative ratio
40
35
30
25
20
15
10
5
0
lung
kidney
pancreas
liver
colon
small intestine
thymus
spleen
heart
coronary arterial endothelium
leukocyte
PHA leukocyte
placenta
uterus
testis
prostate
skeletal muscle
brain

Fig. 2

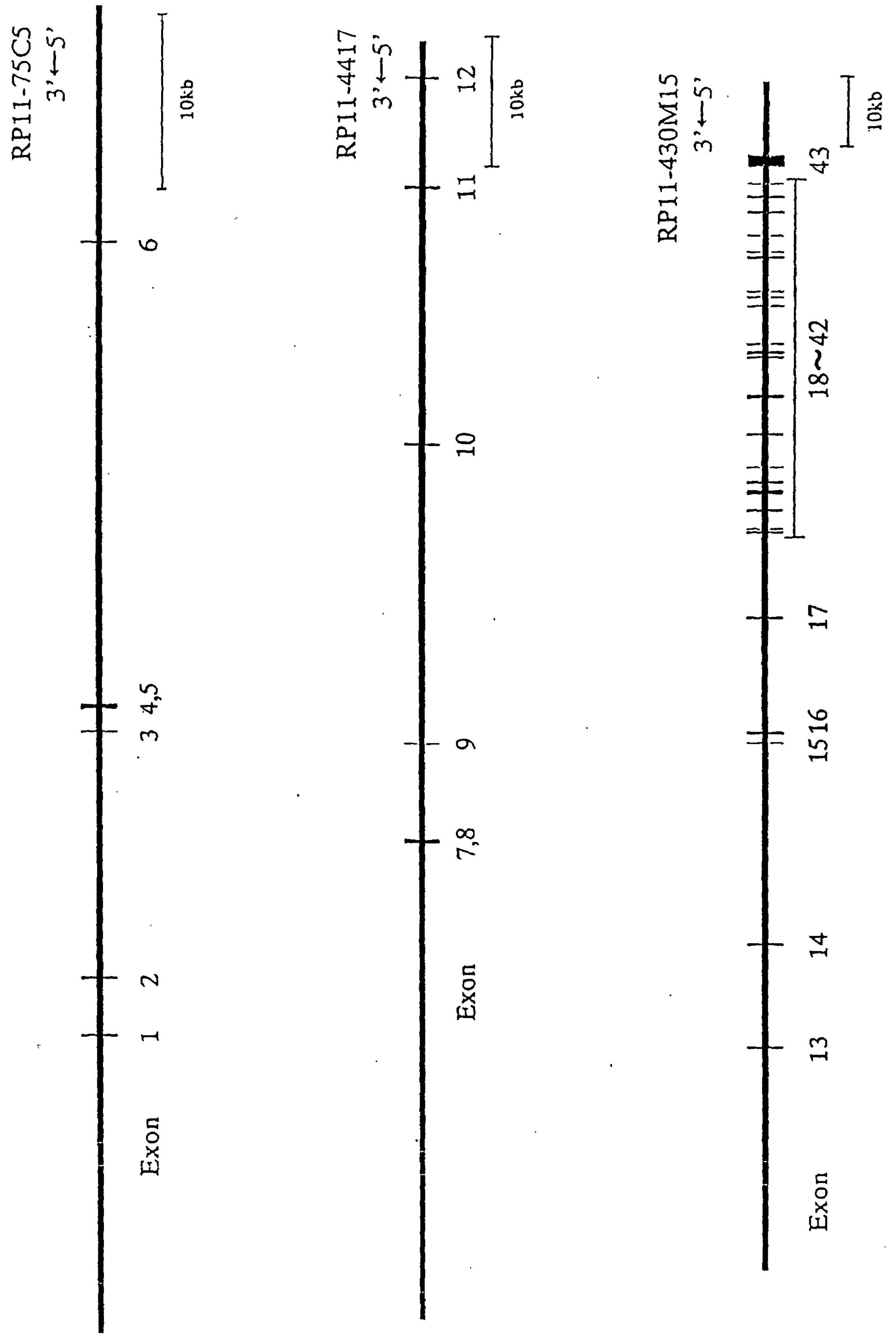
RP11-75C5
3'←5'
10kb
6
3 4,5
2
1
Exon
RP11-4417
3'←5'
10kb
12
11
10
9
7,8
Exon
RP11-430M15
3'←5'
10kb
43
18~42
17
1516
14
13
Exon

Fig. 3-1

I-V plot

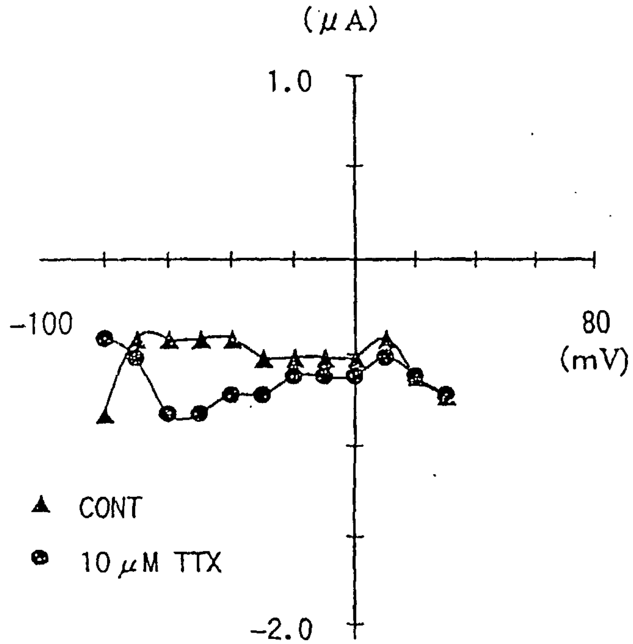


representative figure

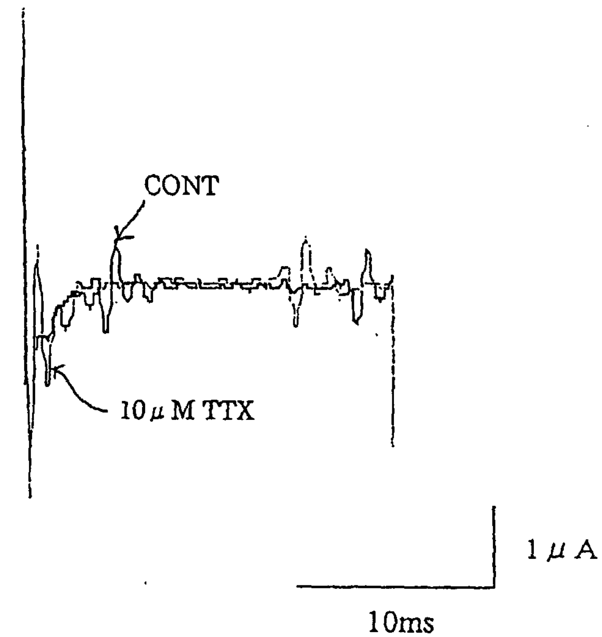


Determination result in CAE9577 (Na current)

amount of RNA introduced: 20 ng/cell
incubation period: 72 hours
extracellular fluid for measurement: 100 mM NaOH, 100 mM methanesulfonic acid, 2 mM $CaCl_2$, 10 mM HEPES, pH 7.3
holding potential: -100 mV
depolarizing stimulus: 20 ms
representative figure: wave form when depolarizing stimulus of -20 mV was applied
I-V plot: current value at the peak (after about 1 ms)
TTX: Na channel inhibitor

Fig. 3-2

I-V plot

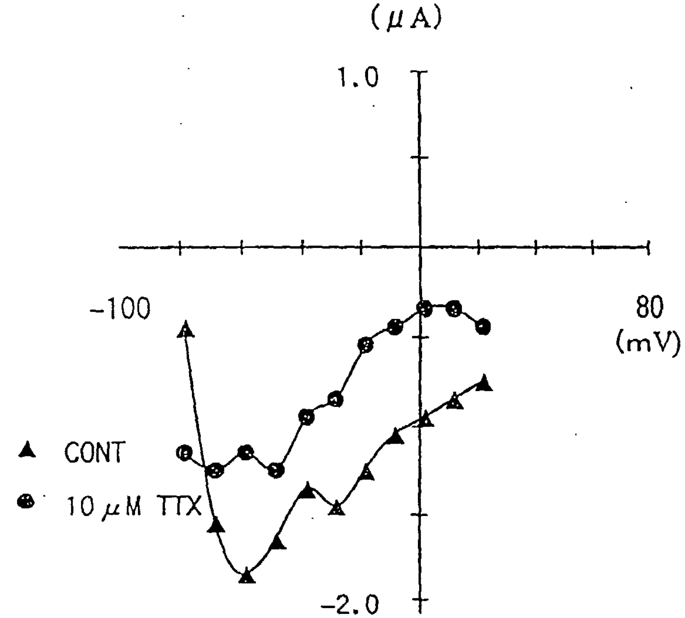


representative figure

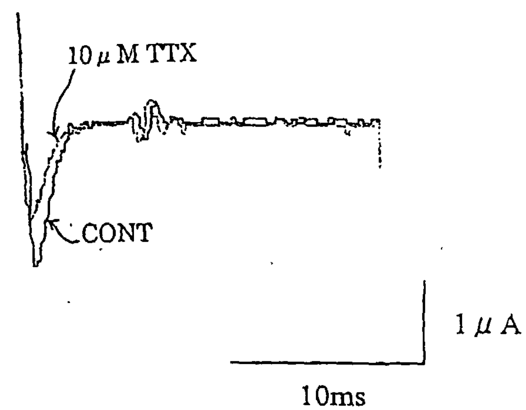


Determination result in CAE9577 + SK-N-SH mRNA (Na current)

amount of RNA introduced: 20 ng/cell
incubation time: 72 hours
extracellular fluid for measurement: 100 mM NaOH, 100 mM methanesulfonic acid, 2 mM CaCl2, 10 mM HEPES, pH 7.3
holding potential: -100 mV
depolarizing stimulus: 20 ms
representative figure: wave form when depolarizing stimulus of -20 mV was applied
I-V plot: current value at the peak (after about 1 ms)
TTX: Na channel inhibitor

Fig. 3-3

I-V plot

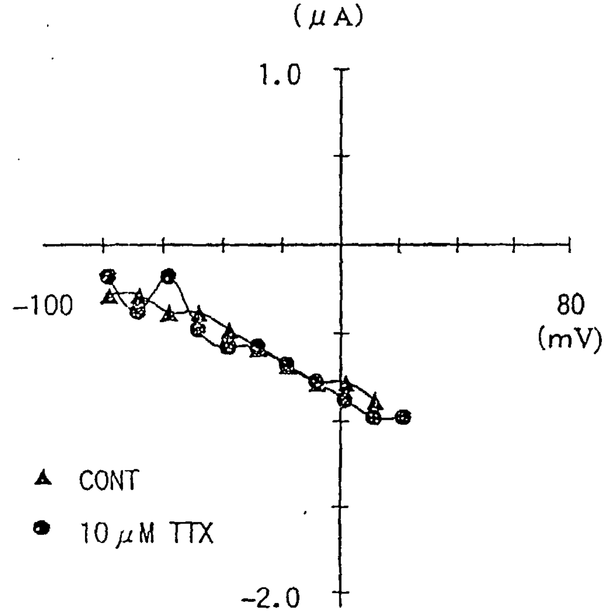


representative figure

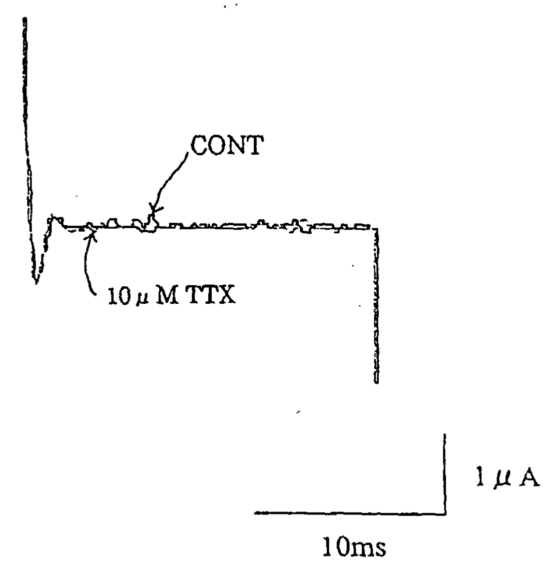


Determination result in SK-N-SH mRNA (Na current)

amount of RNA introduced: 20 ng/cell
incubation period: 72 hours
extracellular fluid for measurement: 100 mM NaOH, 100 mM methanesulfonic acid, 2 mM CaCl2, 10 mM HEPES, pH 7.3
holding potential: -100 mV
depolarizing stimulus: 20 ms
representative figure: wave form when depolarizing stimulus of -20 mV was applied
I-V plot: current value at the peak (after about 1 ms)
TTX: Na channel inhibitor

Fig. 3-4

I-V plot

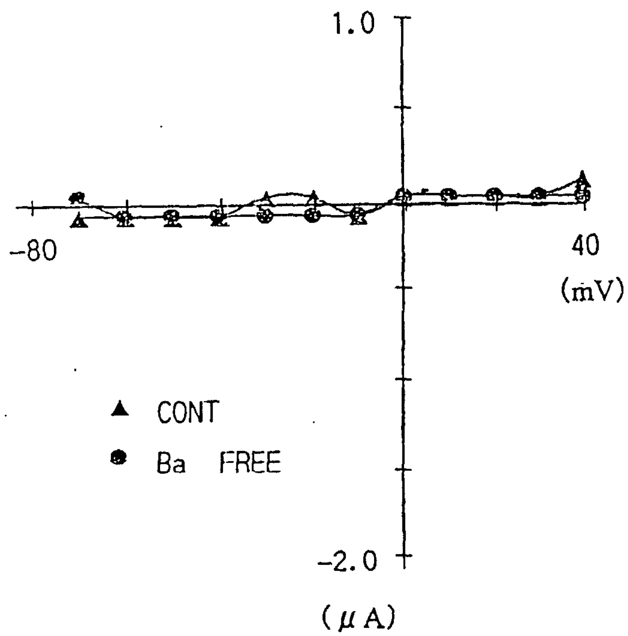


representative figure

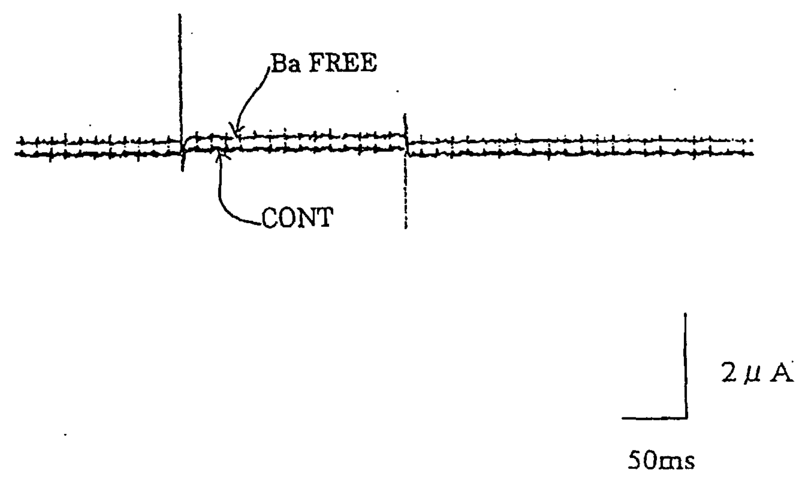


Determination result in CAE9577 (Ca current)

amount of RNA introduced: 20 ng/cell
incubation period: 72 hours
extracellular fluid for measurement: 50 mM NaCl, 2 mM KCl, 40 mM BaCl2, 5 mM HEPES, pH 7.5
In case of Ba FREE, Ba was replaced with Na
holding potential: -80 mV
depolarizing stimulus: 200 ms
representative figure: wave form when depolarizing stimulus of -20 mV was applied

Fig. 3-5

I-V plot

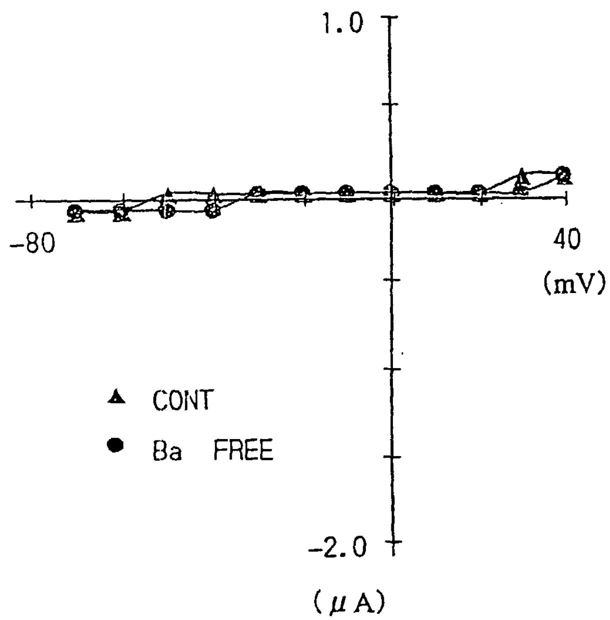


representative figure

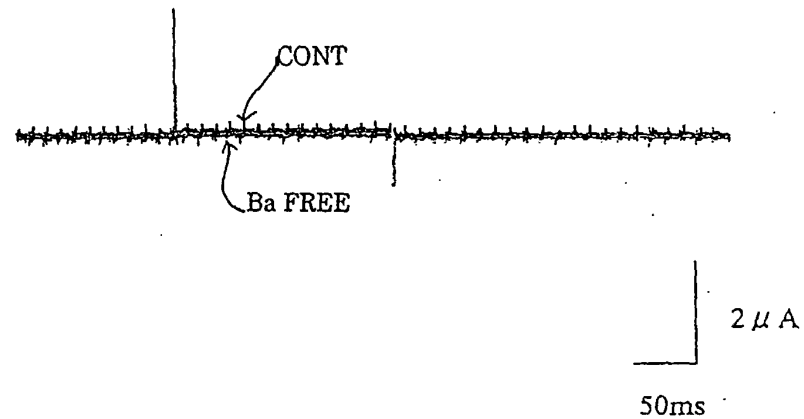


Determination result in CAE9577 + SK-N-SH mRNA (Ca current)

amount of RNA introduced: 20 ng/cell
incubation period: 72 hours
extracellular fluid for measurement: 50 mM NaCl, 2 mM KCl, 40 mM BaCl2, 5 mM HEPES, pH 7.5
In case of Ba FREE, Ba was replaced with Na
holding potential: -80 mV
depolarizing stimulus: 200 ms
representative figure: wave form when depolarizing stimulus of -20 mV was applied

Fig. 3-6

I-V plot

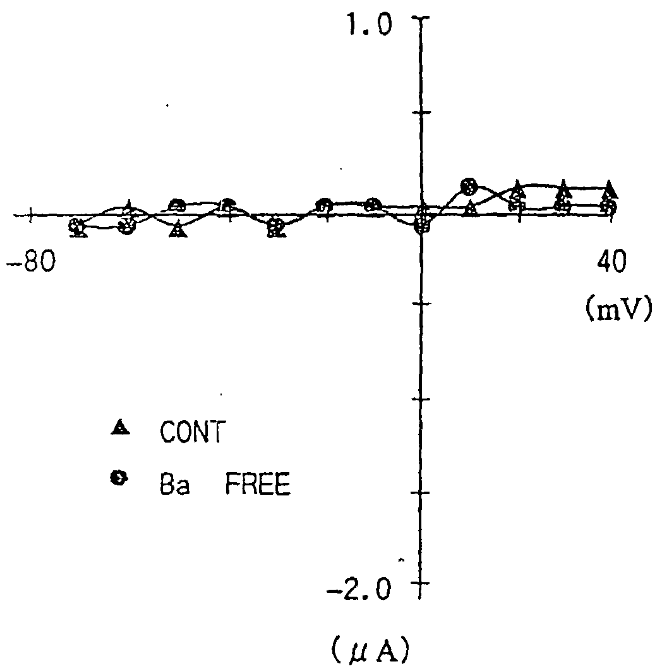


representative figure

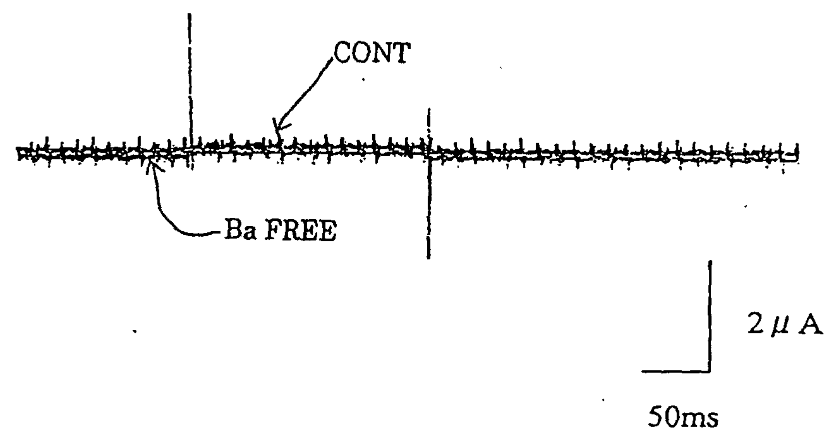


Determination result in SK-N-SH mRNA (Ca current)

amount of RNA introduced: 20 ng/cell
incubation period: 72 hours
extracellular fluid for measurement: 50 mM NaCl, 2 mM KCl, 40 mM BaCl2, 5 mM HEPES, pH 7.5
In case of Ba FREE, Ba was replaced with Na
holding potential: -80 mV
depolarizing stimulus: 200 ms
representative figure: wave form when depolarizing stimulus of -20 mV was applied

## INTERNATIONAL SEARCH REPORT

International application No.
PCT/JP02/04570

**A. CLASSIFICATION OF SUBJECT MATTER**

Int.Cl[7] C12N15/09, C07K14/705, C07K16/28, C12N5/10, C12Q1/02, A61K45/00, A01K67/027

According to International Patent Classification (IPC) or to both national classification and IPC

**B. FIELDS SEARCHED**

Minimum documentation searched (classification system followed by classification symbols)

Int.Cl[7] C12N15/09, C07K14/705, C07K16/28

Documentation searched other than minimum documentation to the extent that such documents are included in the fields searched

Electronic data base consulted during the international search (name of data base and, where practicable, search terms used)

MEDLINE(STN), WPI(DIALOG), BIOSIS(DIALOG), JICST FILE(JOIS), GenBank/EMBL/DDBJ/ GeneSeq, SwissProt/PIR/GeneSeq

**C. DOCUMENTS CONSIDERED TO BE RELEVANT**

| Category* | Citation of document, with indication, where appropriate, of the relevant passages | Relevant to claim No. |
|---|---|---|
| X | LEE, JH. et al., Cloning of a novel four repeat protein related to voltage-gated sodium and calcium channels FEBS Letters(1999), Vol.445, pages 231 to 236 | 1-16 |
| P,X | WO 01/83752 A2 (Millennium Pharm Inc.), 08 November, 2001 (08.11.01), Full text & AU 200157378 A | 1-16 |
| A | WO 99/29847 A1 (Univ. Loyola Chicago), 17 June, 1999 (17.06.99), Full text & EP 1036170 A1 | 1-16 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 July, 2002 (10.07.02) | Date of mailing of the international search report<br>23 July, 2002 (23.07.02) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/04570

C (Continuation). DOCUMENTS CONSIDERED TO BE RELEVANT

| Category* | Citation of document, with indication, where appropriate, of the relevant passages | Relevant to claim No. |
|---|---|---|
| A | SANGAMESWARAN, L. et al., A Novel Tetrodotoxin-sensitive, Voltage-gated Sodium Channel Expressed in Rat and Human Dorsal Root Ganglia, J. Biol. Chem.(1997), Vol.272, No.23, pages 14805 to 14809 | 1-16 |
| A | BURBIDGE, SA. et al, Cloning and expression of the human brain type VI voltage-gated NA+ channel in a mammalian cell line. J. Physiol.(1998), 513Pp130P | 1-16 |
| A | ERTEL EA. et al., Effects of Ni2+ on Cav3.2(alpha1H), a low voltage-activated Ca2+ channel cloned from human heart. Biophys. J.(2000), Vol.78(1 part 2), p203A | 1-16 |

Form PCT/ISA/210 (continuation of second sheet) (July 1998)

SEQUENCE LISTING

<110> Mochida Pharmaceutical Co., Ltd.

<120> Novel Cation channel protein

<130> SAP-686-PCT

<150> JP 2001140690

<151> 2001-05-10

<160> 6

<170> PatentIn Ver. 2.1

<210> 1

<211> 5214

<212> DNA

<213> Homo sapiens

<400> 1

atgctcaaaa ggaagcagag ttccagggtg gaagcccagc cagtcactga ctttggtcct 60
gatgagtctc tgtcggataa tgctgacatc ctctggatta acaaaccatg ggttcactct 120
ttgctgcgca tctgtgccat catcagcgtc atttctgttt gtatgaatac gccaatgacc 180
ttcgagcact atcctccact tcagtatgtg accttcactt tggatacatt attgatgttt 240
ctctacacgg cagagatgat agcaaaaatg cacatccggg gcattgtcaa gggggatagt 300
tcctatgtga aagatcgctg gtgtgttttt gatggattta tggtcttttg cctttgggtt 360
tctttggtgc tacaggtgtt tgaaattgct gatatagttg atcagatgtc accttggggc 420
atgttgcgga ttccacggcc actgattatg atccgagcat tccggattta tttccgattt 480
gaactgccaa ggaccagaat tacaaatatt ttaaagcgat cgggagaaca aatatggagt 540
gtttccattt ttctactttt ctttctactt ctttatggaa ttttaggagt tcagatgttt 600
ggaacattta cttatcactg tgttgtaaat gacacaaagc cagggaatgt aacctggaat 660
agtttagcta ttccagacac acactgctca ccagagctag aagaaggcta ccagtgccca 720
cctggattta aatgcatgga ccttgaagat ctgggactta gcaggcaaga gctgggctac 780
agtggcttta atgagatagg aactagtata ttcaccgtct atgaggccgc ctcacaggaa 840
ggctgggtgt tcctcatgta cagagcaatt gacagctttc cccgttggcg ttcctacttc 900
tatttcatca ctctcatttt cttcctcgcc tggcttgtga agaacgtgtt tattgctgtt 960
atcattgaaa catttgcaga aatcagagta cagtttcaac aaatgtgggg atcgagaagc 1020
agcactacct caacagccac cacccagatg tttcatgaag atgctgctgg aggttggcag 1080
ctggtagctg tggatgtcaa caagccccag ggacgcgccc cagcctgcct ccagaaaatg 1140

atgcggtcat ccgttttcca catgttcatc ctgagcatgg tgaccgtgga cgtgatcgtg 1200
gcggctagca actactacaa aggagaaaac ttcaggaggc agtacgacga gttctacctg 1260
gcggaggtgg cttttacagt actttttgat ttggaagcac ttctgaagat atggtgtttg 1320
ggatttactg gatatattag ctcatctctc cacaaattcg aactactact cgtaattgga 1380
actactcttc atgtataccc agatctttat cattcacaat tcacgtactt tcaggttctc 1440
cgagtagttc ggctgattaa gatttcacct gcattagaag actttgtgta caagatattt 1500
ggtcctggaa aaaagcttgg gagtttggtt gtatttactg ccagcctctt gattgttatg 1560
tcagcaatta gtttgcagat gttctgcttt gtcgaagaac tggacagatt tactacgttt 1620
ccgagggcat ttatgtccat gttccagatc ctcacccagg aaggatgggt ggacgtaatg 1680
gaccaaactc taaatgctgt gggacatatg tgggcacccg tggttgccat ctatttcatt 1740
ctctatcatc tttttgccac tctgatcctc ctgagtttgt ttgttgctgt tattttggac 1800
aacttagaac ttgatgaaga cctaaagaag cttaaacaat taaagcaaag tgaagcaaat 1860
gcggacacca aagaaaagct ccctttacgc ctgcgaatct ttgaaaaatt tccaaacaga 1920
cctcaaatgg tgaaaatctc aaagcttcct tcagatttta cagttcctaa aatcagggag 1980
agttttatga agcagtttat tgaccgccag caacaggaca catgttgcct cctgagaagc 2040
ctcccgacca cctcttcctc ctcctgcgac cactccaaac gctcagcaat tgaggacaac 2100
aaatacatcg accaaaaact tcgcaagtct gttttcagca tcagggcaag gaaccttctg 2160
gaaaaggaga ccgcagtcac taaaatctta agagcttgca cccgacagcg catgctgagc 2220
ggatcatttg aggggcagcc cgcaaaggag aggtcaatcc tcagcgtgca gcatcatatc 2280
cgccaagagc gcaggtcact aagacatgga tcaaacagcc agaggatcag caggggaaaa 2340
tctcttgaaa ctttgactca agatcattcc aatacagtga gatatagaaa tgcacaaaga 2400
gaagacagtg aaataaagat gattcaggaa aaaaaggagc aagcagagat gaaaaggaaa 2460
gtgcaagaag aggaactcag agagaaccac ccatacttcg ataagccact gttcattgtc 2520
gggcgagaac acaggttcag aaacttttgc cgggtggtgg tccgagcacg cttcaacgca 2580
tctaaaacag accctgtcac aggagctgtg aaaaatacaa agtaccatca actttatgat 2640
ttgctgggat tggtcactta cctggactgg gtcatgatca tcgtaaccat ctgctcttgc 2700
atttccatga tgtttgagtc cccgtttcga agagtcatgc atgcacctac tttgcagatt 2760
gctgagtatg tgtttgtgat attcatgagc attgagctta atctgaagat tatggcagat 2820
ggcttatttt tcactccaac tgctgtcatc agggacttcg gtggagtaat ggacatattt 2880
atatatcttg tgagcttgat atttctttgt tggatgcctc aaaatgtacc tgctgaatcg 2940
ggagctcagc ttctaatggt ccttcggtgc ctgagacctc tgcgcatatt caaactggtg 3000
ccccagatga ggaaagttgt tcgagaactt ttcagcggct tcaaggaaat ttttttggtc 3060
tccattcttt tgctgacatt aatgctcgtt tttgcaagct ttggagttca gctttttgct 3120
ggaaaactgg ccaagtgcaa tgatcccaac attattagaa gggaagattg caatggcata 3180
ttcagaatta atgtcagtgt gtcaaagaac ttaaatttaa aattgaggcc tggagagaaa 3240
aaacctggat tttgggtgcc ccgtgtttgg gcgaatcctc ggaactttaa tttcgacaat 3300

gtgggaaacg ctatgctggc gttgtttgaa gttctctcct tgaaaggctg ggtggaagtg 3360
agagatgtta ttattcatcg tgtggggccg atccatggaa tctatattca tgtttttgta 3420
ttcctgggtt gcatgattgg actgaccctt tttgttggag tagttattgc taatttcaat 3480
gaaaacaagg ggacggcttt gctgaccgtc gatcagagaa gatgggaaga cctgaagagc 3540
cgactgaaga tcgcacagcc tcttcatctt ccgcctcgcc cggataatga tggttttaga 3600
gctaaaatgt atgacataac ccagcatcca tttttaaga ggacaatcgc attactcgtc 3660
ctggcccagt cggtgttgct ctctgtcaag tgggacgtcg aggacccggt gaccgtacct 3720
ttggcaacaa tgtcagttgt tttcaccttc atctttgttc tggaggttac catgaagatc 3780
atagcaatgt cgcctgctgg cttctggcaa agcagaagaa accgatacga tctcctggtg 3840
acgtcgcttg gcgttgtatg ggtggtgctt cactttgccc tcctgaatgc atatacttac 3900
atgatgggcg cttgtgtgat tgtatttagg tttttctcca tctgtggaaa acatgtaacg 3960
ctaaagatgc tcctcttgac agtggtcgtc agcatgtaca agagcttctt tatcatagta 4020
ggcatgtttc tcttgctgct gtgttacgct tttgctggag ttgttttatt tggtactgtg 4080
aaatatgggg agaatattaa caggcatgca aatttttctt cggctggaaa agctattacc 4140
gtactgttcc gaattgtcac aggtgaagac tggaacaaga ttatgcatga ctgtatggtt 4200
cagcctccgt tttgtactcc agatgaattt acatactggg caacagactg tggaaattat 4260
gctggggcac ttatgtattt ctgttcattt tatgtcatca ttgcctacat catgctaaat 4320
ctgcttgtag ccataattgt ggagaatttc tccttgtttt attccactga ggaggaccag 4380
cttttaagtt acaatgatct tcgccacttt caaatcatat ggaacatggt ggatgataaa 4440
agagaggggg tgatccccac gttccgcgtc aagttcctgc tgcggctact gcgtgggagg 4500
ctggaggtgg acctggacaa ggacaagctc ctgtttaagc acatgtgcta cgaaatggag 4560
aggctccaca atggcggcga cgtcaccttc catgatgtcc tgagcatgct ttcataccgg 4620
tccgtggaca tccggaagag cttgcagctg gaggaactcc tggcgaggga gcagctggag 4680
tacaccatag aggaggaggt ggccaagcag accatccgca tgtggctcaa gaagtgcctg 4740
aagcgcatca gagctaaaca gcagcagtcg tgcagtatca tccacagcct gagagagagt 4800
cagcagcaag agctgagccg gtttctgaac ccgcccagca tcgagaccac ccagcccagt 4860
gaggacacga atgccaacag tcaggacaac agcatgcaac ctgagacaag cagccagcag 4920
cagctcctga gccccacgct gtcggatcga ggaggaagtc ggcaagatgc agccgacgca 4980
gggaaacccc agaggaaatt tgggcagtgg cgtctgccct cagccccaaa accaataagc 5040
cattcagtgt cctcagtcaa cttacggttt ggaggaagga caaccatgaa atctgtcgtg 5100
tgcaaaatga accccatgac tgacgcggct tcctgcggtt ctgaagttaa gaagtggtgg 5160
acccggcagc tgactgtgga gagcgacgaa agtggggatg accttctgga tatt 5214

<210> 2
<211> 1738
<212> PRT

<213> Homo sapiens

<400> 2

```
Met Leu Lys Arg Lys Gln Ser Ser Arg Val Glu Ala Gln Pro Val Thr
 1               5                  10                  15
Asp Phe Gly Pro Asp Glu Ser Leu Ser Asp Asn Ala Asp Ile Leu Trp
            20                  25                  30
Ile Asn Lys Pro Trp Val His Ser Leu Leu Arg Ile Cys Ala Ile Ile
        35                  40                  45
Ser Val Ile Ser Val Cys Met Asn Thr Pro Met Thr Phe Glu His Tyr
    50                  55                  60
Pro Pro Leu Gln Tyr Val Thr Phe Thr Leu Asp Thr Leu Leu Met Phe
65                  70                  75                  80
Leu Tyr Thr Ala Glu Met Ile Ala Lys Met His Ile Arg Gly Ile Val
                85                  90                  95
Lys Gly Asp Ser Ser Tyr Val Lys Asp Arg Trp Cys Val Phe Asp Gly
            100                 105                 110
Phe Met Val Phe Cys Leu Trp Val Ser Leu Val Leu Gln Val Phe Glu
        115                 120                 125
Ile Ala Asp Ile Val Asp Gln Met Ser Pro Trp Gly Met Leu Arg Ile
    130                 135                 140
Pro Arg Pro Leu Ile Met Ile Arg Ala Phe Arg Ile Tyr Phe Arg Phe
145                 150                 155                 160
Glu Leu Pro Arg Thr Arg Ile Thr Asn Ile Leu Lys Arg Ser Gly Glu
                165                 170                 175
Gln Ile Trp Ser Val Ser Ile Phe Leu Leu Phe Phe Leu Leu Leu Tyr
            180                 185                 190
Gly Ile Leu Gly Val Gln Met Phe Gly Thr Phe Thr Tyr His Cys Val
        195                 200                 205
Val Asn Asp Thr Lys Pro Gly Asn Val Thr Trp Asn Ser Leu Ala Ile
    210                 215                 220
Pro Asp Thr His Cys Ser Pro Glu Leu Glu Glu Gly Tyr Gln Cys Pro
225                 230                 235                 240
Pro Gly Phe Lys Cys Met Asp Leu Glu Asp Leu Gly Leu Ser Arg Gln
                245                 250                 255
Glu Leu Gly Tyr Ser Gly Phe Asn Glu Ile Gly Thr Ser Ile Phe Thr
```

```
        260                 265                 270
Val Tyr Glu Ala Ala Ser Gln Glu Gly Trp Val Phe Leu Met Tyr Arg
    275                 280                 285
Ala Ile Asp Ser Phe Pro Arg Trp Arg Ser Tyr Phe Tyr Phe Ile Thr
    290                 295                 300
Leu Ile Phe Phe Leu Ala Trp Leu Val Lys Asn Val Phe Ile Ala Val
305                 310                 315                 320
Ile Ile Glu Thr Phe Ala Glu Ile Arg Val Gln Phe Gln Gln Met Trp
                325                 330                 335
Gly Ser Arg Ser Ser Thr Thr Ser Thr Ala Thr Thr Gln Met Phe His
            340                 345                 350
Glu Asp Ala Ala Gly Gly Trp Gln Leu Val Ala Val Asp Val Asn Lys
        355                 360                 365
Pro Gln Gly Arg Ala Pro Ala Cys Leu Gln Lys Met Met Arg Ser Ser
    370                 375                 380
Val Phe His Met Phe Ile Leu Ser Met Val Thr Val Asp Val Ile Val
385                 390                 395                 400
Ala Ala Ser Asn Tyr Tyr Lys Gly Glu Asn Phe Arg Arg Gln Tyr Asp
                405                 410                 415
Glu Phe Tyr Leu Ala Glu Val Ala Phe Thr Val Leu Phe Asp Leu Glu
            420                 425                 430
Ala Leu Leu Lys Ile Trp Cys Leu Gly Phe Thr Gly Tyr Ile Ser Ser
        435                 440                 445
Ser Leu His Lys Phe Glu Leu Leu Leu Val Ile Gly Thr Thr Leu His
    450                 455                 460
Val Tyr Pro Asp Leu Tyr His Ser Gln Phe Thr Tyr Phe Gln Val Leu
465                 470                 475                 480
Arg Val Val Arg Leu Ile Lys Ile Ser Pro Ala Leu Glu Asp Phe Val
                485                 490                 495
Tyr Lys Ile Phe Gly Pro Gly Lys Lys Leu Gly Ser Leu Val Val Phe
            500                 505                 510
Thr Ala Ser Leu Leu Ile Val Met Ser Ala Ile Ser Leu Gln Met Phe
        515                 520                 525
Cys Phe Val Glu Glu Leu Asp Arg Phe Thr Thr Phe Pro Arg Ala Phe
    530                 535                 540
Met Ser Met Phe Gln Ile Leu Thr Gln Glu Gly Trp Val Asp Val Met
```

```
545                 550                 555                 560
Asp Gln Thr Leu Asn Ala Val Gly His Met Trp Ala Pro Val Val Ala
                565                 570                 575
Ile Tyr Phe Ile Leu Tyr His Leu Phe Ala Thr Leu Ile Leu Leu Ser
            580                 585                 590
Leu Phe Val Ala Val Ile Leu Asp Asn Leu Glu Leu Asp Glu Asp Leu
        595                 600                 605
Lys Lys Leu Lys Gln Leu Lys Gln Ser Glu Ala Asn Ala Asp Thr Lys
    610                 615                 620
Glu Lys Leu Pro Leu Arg Leu Arg Ile Phe Glu Lys Phe Pro Asn Arg
625                 630                 635                 640
Pro Gln Met Val Lys Ile Ser Lys Leu Pro Ser Asp Phe Thr Val Pro
                645                 650                 655
Lys Ile Arg Glu Ser Phe Met Lys Gln Phe Ile Asp Arg Gln Gln Gln
            660                 665                 670
Asp Thr Cys Cys Leu Leu Arg Ser Leu Pro Thr Thr Ser Ser Ser Ser
        675                 680                 685
Cys Asp His Ser Lys Arg Ser Ala Ile Glu Asp Asn Lys Tyr Ile Asp
    690                 695                 700
Gln Lys Leu Arg Lys Ser Val Phe Ser Ile Arg Ala Arg Asn Leu Leu
705                 710                 715                 720
Glu Lys Glu Thr Ala Val Thr Lys Ile Leu Arg Ala Cys Thr Arg Gln
                725                 730                 735
Arg Met Leu Ser Gly Ser Phe Glu Gly Gln Pro Ala Lys Glu Arg Ser
            740                 745                 750
Ile Leu Ser Val Gln His His Ile Arg Gln Glu Arg Arg Ser Leu Arg
        755                 760                 765
His Gly Ser Asn Ser Gln Arg Ile Ser Arg Gly Lys Ser Leu Glu Thr
    770                 775                 780
Leu Thr Gln Asp His Ser Asn Thr Val Arg Tyr Arg Asn Ala Gln Arg
785                 790                 795                 800
Glu Asp Ser Glu Ile Lys Met Ile Gln Glu Lys Lys Glu Gln Ala Glu
                805                 810                 815
Met Lys Arg Lys Val Gln Glu Glu Glu Leu Arg Glu Asn His Pro Tyr
            820                 825                 830
Phe Asp Lys Pro Leu Phe Ile Val Gly Arg Glu His Arg Phe Arg Asn
```

835 840 845

Phe Cys Arg Val Val Val Arg Ala Arg Phe Asn Ala Ser Lys Thr Asp

850 855 860

Pro Val Thr Gly Ala Val Lys Asn Thr Lys Tyr His Gln Leu Tyr Asp

865 870 875 880

Leu Leu Gly Leu Val Thr Tyr Leu Asp Trp Val Met Ile Ile Val Thr

885 890 895

Ile Cys Ser Cys Ile Ser Met Met Phe Glu Ser Pro Phe Arg Arg Val

900 905 910

Met His Ala Pro Thr Leu Gln Ile Ala Glu Tyr Val Phe Val Ile Phe

915 920 925

Met Ser Ile Glu Leu Asn Leu Lys Ile Met Ala Asp Gly Leu Phe Phe

930 935 940

Thr Pro Thr Ala Val Ile Arg Asp Phe Gly Gly Val Met Asp Ile Phe

945 950 955 960

Ile Tyr Leu Val Ser Leu Ile Phe Leu Cys Trp Met Pro Gln Asn Val

965 970 975

Pro Ala Glu Ser Gly Ala Gln Leu Leu Met Val Leu Arg Cys Leu Arg

980 985 990

Pro Leu Arg Ile Phe Lys Leu Val Pro Gln Met Arg Lys Val Val Arg

995 1000 1005

Glu Leu Phe Ser Gly Phe Lys Glu Ile Phe Leu Val Ser Ile Leu Leu

1010 1015 1020

Leu Thr Leu Met Leu Val Phe Ala Ser Phe Gly Val Gln Leu Phe Ala

1025 1030 1035 1040

Gly Lys Leu Ala Lys Cys Asn Asp Pro Asn Ile Ile Arg Arg Glu Asp

1045 1050 1055

Cys Asn Gly Ile Phe Arg Ile Asn Val Ser Val Ser Lys Asn Leu Asn

1060 1065 1070

Leu Lys Leu Arg Pro Gly Glu Lys Lys Pro Gly Phe Trp Val Pro Arg

1075 1080 1085

Val Trp Ala Asn Pro Arg Asn Phe Asn Phe Asp Asn Val Gly Asn Ala

1090 1095 1100

Met Leu Ala Leu Phe Glu Val Leu Ser Leu Lys Gly Trp Val Glu Val

1105 1110 1115 1120

Arg Asp Val Ile Ile His Arg Val Gly Pro Ile His Gly Ile Tyr Ile

1125 1130 1135

His Val Phe Val Phe Leu Gly Cys Met Ile Gly Leu Thr Leu Phe Val

1140 1145 1150

Gly Val Val Ile Ala Asn Phe Asn Glu Asn Lys Gly Thr Ala Leu Leu

1155 1160 1165

Thr Val Asp Gln Arg Arg Trp Glu Asp Leu Lys Ser Arg Leu Lys Ile

1170 1175 1180

Ala Gln Pro Leu His Leu Pro Pro Arg Pro Asp Asn Asp Gly Phe Arg

1185 1190 1195 1200

Ala Lys Met Tyr Asp Ile Thr Gln His Pro Phe Phe Lys Arg Thr Ile

1205 1210 1215

Ala Leu Leu Val Leu Ala Gln Ser Val Leu Leu Ser Val Lys Trp Asp

1220 1225 1230

Val Glu Asp Pro Val Thr Val Pro Leu Ala Thr Met Ser Val Val Phe

1235 1240 1245

Thr Phe Ile Phe Val Leu Glu Val Thr Met Lys Ile Ile Ala Met Ser

1250 1255 1260

Pro Ala Gly Phe Trp Gln Ser Arg Arg Asn Arg Tyr Asp Leu Leu Val

1265 1270 1275 1280

Thr Ser Leu Gly Val Val Trp Val Val Leu His Phe Ala Leu Leu Asn

1285 1290 1295

Ala Tyr Thr Tyr Met Met Gly Ala Cys Val Ile Val Phe Arg Phe Phe

1300 1305 1310

Ser Ile Cys Gly Lys His Val Thr Leu Lys Met Leu Leu Leu Thr Val

1315 1320 1325

Val Val Ser Met Tyr Lys Ser Phe Phe Ile Ile Val Gly Met Phe Leu

1330 1335 1340

Leu Leu Leu Cys Tyr Ala Phe Ala Gly Val Val Leu Phe Gly Thr Val

1345 1350 1355 1360

Lys Tyr Gly Glu Asn Ile Asn Arg His Ala Asn Phe Ser Ser Ala Gly

1365 1370 1375

Lys Ala Ile Thr Val Leu Phe Arg Ile Val Thr Gly Glu Asp Trp Asn

1380 1385 1390

Lys Ile Met His Asp Cys Met Val Gln Pro Pro Phe Cys Thr Pro Asp

1395 1400 1405

Glu Phe Thr Tyr Trp Ala Thr Asp Cys Gly Asn Tyr Ala Gly Ala Leu

1410 1415 1420

Met Tyr Phe Cys Ser Phe Tyr Val Ile Ile Ala Tyr Ile Met Leu Asn
1425 1430 1435 1440

Leu Leu Val Ala Ile Ile Val Glu Asn Phe Ser Leu Phe Tyr Ser Thr
1445 1450 1455

Glu Glu Asp Gln Leu Leu Ser Tyr Asn Asp Leu Arg His Phe Gln Ile
1460 1465 1470

Ile Trp Asn Met Val Asp Asp Lys Arg Glu Gly Val Ile Pro Thr Phe
1475 1480 1485

Arg Val Lys Phe Leu Leu Arg Leu Leu Arg Gly Arg Leu Glu Val Asp
1490 1495 1500

Leu Asp Lys Asp Lys Leu Leu Phe Lys His Met Cys Tyr Glu Met Glu
1505 1510 1515 1520

Arg Leu His Asn Gly Gly Asp Val Thr Phe His Asp Val Leu Ser Met
1525 1530 1535

Leu Ser Tyr Arg Ser Val Asp Ile Arg Lys Ser Leu Gln Leu Glu Glu
1540 1545 1550

Leu Leu Ala Arg Glu Gln Leu Glu Tyr Thr Ile Glu Glu Glu Val Ala
1555 1560 1565

Lys Gln Thr Ile Arg Met Trp Leu Lys Lys Cys Leu Lys Arg Ile Arg
1570 1575 1580

Ala Lys Gln Gln Gln Ser Cys Ser Ile Ile His Ser Leu Arg Glu Ser
1585 1590 1595 1600

Gln Gln Gln Glu Leu Ser Arg Phe Leu Asn Pro Pro Ser Ile Glu Thr
1605 1610 1615

Thr Gln Pro Ser Glu Asp Thr Asn Ala Asn Ser Gln Asp Asn Ser Met
1620 1625 1630

Gln Pro Glu Thr Ser Ser Gln Gln Gln Leu Leu Ser Pro Thr Leu Ser
1635 1640 1645

Asp Arg Gly Gly Ser Arg Gln Asp Ala Ala Asp Ala Gly Lys Pro Gln
1650 1655 1660

Arg Lys Phe Gly Gln Trp Arg Leu Pro Ser Ala Pro Lys Pro Ile Ser
1665 1670 1675 1680

His Ser Val Ser Ser Val Asn Leu Arg Phe Gly Gly Arg Thr Thr Met
1685 1690 1695

Lys Ser Val Val Cys Lys Met Asn Pro Met Thr Asp Ala Ala Ser Cys

1700 1705 1710

Gly Ser Glu Val Lys Lys Trp Trp Thr Arg Gln Leu Thr Val Glu Ser

1715 1720 1725

Asp Glu Ser Gly Asp Asp Leu Leu Asp Ile

1730 1735

<210> 3

<211> 6789

<212> DNA

<213> Homo sapiens

<400> 3

ctgtggtttt gtgcttgctc accaaagcta acctcagcat gctcaaaagg aagcagagtt 60
ccagggtgga agcccagcca gtcactgact ttggtcctga tgagtctctg tcggataatg 120
ctgacatcct ctggattaac aaaccatggg ttcactcttt gctgcgcatc tgtgccatca 180
tcagcgtcat ttctgtttgt atgaatacgc caatgacctt cgagcactat cctccacttc 240
agtatgtgac cttcactttg gatacattat tgatgtttct ctacacggca gagatgatag 300
caaaaatgca catccggggc attgtcaagg gggatagttc ctatgtgaaa gatcgctggt 360
gtgtttttga tggatttatg gtcttttgcc tttgggtttc tttggtgcta caggtgtttg 420
aaattgctga tatagttgat cagatgtcac cttggggcat gttgcggatt ccacggccac 480
tgattatgat ccgagcattc cggatttatt tccgatttga actgccaagg accagaatta 540
caaatatttt aaagcgatcg ggagaacaaa tatggagtgt ttccattttt ctacttttct 600
ttctacttct ttatggaatt ttaggagttc agatgtttgg aacatttact tatcactgtg 660
ttgtaaatga cacaaagcca gggaatgtaa cctggaatag tttagctatt ccagacacac 720
actgctcacc agagctagaa gaaggctacc agtgcccacc tggatttaaa tgcatggacc 780
ttgaagatct gggacttagc aggcaagagc tgggctacag tggctttaat gagataggaa 840
ctagtatatt caccgtctat gaggccgcct cacaggaagg ctgggtgttc ctcatgtaca 900
gagcaattga cagctttccc cgttggcgtt cctacttcta tttcatcact ctcattttct 960
tcctcgcctg gcttgtgaag aacgtgttta ttgctgttat cattgaaaca tttgcagaaa 1020
tcagagtaca gtttcaacaa atgtggggat cgagaagcag cactacctca acagccacca 1080
cccagatgtt tcatgaagat gctgctggag gttggcagct ggtagctgtg gatgtcaaca 1140
agccccaggg acgcgcccca gcctgcctcc agaaaatgat gcggtcatcc gttttccaca 1200
tgttcatcct gagcatggtg accgtggacg tgatcgtggc ggctagcaac tactacaaag 1260
gagaaaactt caggaggcag tacgacgagt tctacctggc ggaggtggct tttacagtac 1320
tttttgattt ggaagcactt ctgaagatat ggtgtttggg atttactgga tatattagct 1380
catctctcca caaattcgaa ctactactcg taattggaac tactcttcat gtatacccag 1440

atctttatca ttcacaattc acgtactttc aggttctccg agtagttcgg ctgattaaga 1500
tttcacctgc attagaagac tttgtgtaca agatatttgg tcctggaaaa aagcttggga 1560
gtttggttgt atttactgcc agcctcttga ttgttatgtc agcaattagt ttgcagatgt 1620
tctgctttgt cgaagaactg gacagattta ctacgtttcc gagggcattt atgtccatgt 1680
tccagatcct cacccaggaa ggatgggtgg acgtaatgga ccaaactcta aatgctgtgg 1740
gacatatgtg ggcacccgtg gttgccatct atttcattct ctatcatctt tttgccactc 1800
tgatcctcct gagtttgttt gttgctgtta ttttggacaa cttagaactt gatgaagacc 1860
taaagaagct taaacaatta aagcaaagtg aagcaaatgc ggacaccaaa gaaaagctcc 1920
ctttacgcct gcgaatcttt gaaaaatttc caaacagacc tcaaatggtg aaaatctcaa 1980
agcttccttc agattttaca gttcctaaaa tcagggagag ttttatgaag cagtttattg 2040
accgccagca acaggacaca tgttgcctcc tgagaagcct cccgaccacc tcttcctcct 2100
cctgcgacca ctccaaacgc tcagcaattg aggacaacaa atacatcgac caaaaacttc 2160
gcaagtctgt tttcagcatc agggcaagga accttctgga aaaggagacc gcagtcacta 2220
aaatcttaag agcttgcacc cgacagcgca tgctgagcgg atcatttgag gggcagcccg 2280
caaaggagag gtcaatcctc agcgtgcagc atcatatccg ccaagagcgc aggtcactaa 2340
gacatggatc aaacagccag aggatcagca ggggaaaatc tcttgaaact ttgactcaag 2400
atcattccaa tacagtgaga tatagaaatg cacaaagaga agacagtgaa ataaagatga 2460
ttcaggaaaa aaaggagcaa gcagagatga aaaggaaagt gcaagaagag gaactcagag 2520
agaaccaccc atacttcgat aagccactgt tcattgtcgg gcgagaacac aggttcagaa 2580
acttttgccg ggtggtggtc cgagcacgct tcaacgcatc taaaacagac cctgtcacag 2640
gagctgtgaa aaatacaaag taccatcaac tttatgattt gctgggattg gtcacttacc 2700
tggactgggt catgatcatc gtaaccatct gctcttgcat ttccatgatg tttgagtccc 2760
cgtttcgaag agtcatgcat gcacctactt tgcagattgc tgagtatgtg tttgtgatat 2820
tcatgagcat tgagcttaat ctgaagatta tggcagatgg cttatttttc actccaactg 2880
ctgtcatcag ggacttcggt ggagtaatgg acatatttat atatcttgtg agcttgatat 2940
ttctttgttg gatgcctcaa aatgtacctg ctgaatcggg agctcagctt ctaatggtcc 3000
ttcggtgcct gagacctctg cgcatattca aactggtgcc ccagatgagg aaagttgttc 3060
gagaactttt cagcggcttc aaggaaattt ttttggtctc cattcttttg ctgacattaa 3120
tgctcgtttt tgcaagcttt ggagttcagc tttttgctgg aaaactggcc aagtgcaatg 3180
atcccaacat tattagaagg gaagattgca atggcatatt cagaattaat gtcagtgtgt 3240
caaagaactt aaatttaaaa ttgaggcctg gagagaaaaa acctggattt tgggtgcccc 3300
gtgtttgggc gaatcctcgg aactttaatt tcgacaatgt gggaaacgct atgctggcgt 3360
tgtttgaagt tctctccttg aaaggctggg tggaagtgag agatgttatt attcatcgtg 3420
tggggccgat ccatggaatc tatattcatg tttttgtatt cctgggttgc atgattggac 3480
tgaccctttt tgttggagta gttattgcta atttcaatga aaacaagggg acggctttgc 3540
tgaccgtcga tcagagaaga tgggaagacc tgaagagccg actgaagatc gcacagcctc 3600

```
ttcatcttcc gcctcgcccg gataatgatg gttttagagc taaaatgtat gacataaccc 3660
agcatccatt ttttaagagg acaatcgcat tactcgtcct ggcccagtcg gtgttgctct 3720
ctgtcaagtg ggacgtcgag gacccggtga ccgtaccttt ggcaacaatg tcagttgttt 3780
tcaccttcat ctttgttctg gaggttacca tgaagatcat agcaatgtcg cctgctggct 3840
tctggcaaag cagaagaaac cgatacgatc tcctggtgac gtcgcttggc gttgtatggg 3900
tggtgcttca ctttgccctc ctgaatgcat atacttacat gatgggcgct tgtgtgattg 3960
tatttaggtt tttctccatc tgtggaaaac atgtaacgct aaagatgctc ctcttgacag 4020
tggtcgtcag catgtacaag agcttcttta tcatagtagg catgtttctc ttgctgctgt 4080
gttacgcttt tgctggagtt gttttatttg gtactgtgaa atatggggag aatattaaca 4140
ggcatgcaaa tttttcttcg gctggaaaag ctattaccgt actgttccga attgtcacag 4200
gtgaagactg gaacaagatt atgcatgact gtatggttca gcctccgttt tgtactccag 4260
atgaatttac atactgggca acagactgtg gaaattatgc tggggcactt atgtatttct 4320
gttcatttta tgtcatcatt gcctacatca tgctaaatct gcttgtagcc ataattgtgg 4380
agaatttctc cttgttttat tccactgagg aggaccagct tttaagttac aatgatcttc 4440
gccactttca aatcatatgg aacatggtgg atgataaaag agagggggtg atccccacgt 4500
tccgcgtcaa gttcctgctg cggctactgc gtgggaggct ggaggtggac ctggacaagg 4560
acaagctcct gtttaagcac atgtgctacg aaatggagag gctccacaat ggcggcgacg 4620
tcaccttcca tgatgtcctg agcatgcttt cataccggtc cgtggacatc cggaagagct 4680
tgcagctgga ggaactcctg gcgagggagc agctggagta caccatagag gaggaggtgg 4740
ccaagcagac catccgcatg tggctcaaga agtgcctgaa gcgcatcaga gctaaacagc 4800
agcagtcgtg cagtatcatc cacagcctga gagagagtca gcagcaagag ctgagccggt 4860
ttctgaaccc gcccagcatc gagaccaccc agcccagtga ggacacgaat gccaacagtc 4920
aggacaacag catgcaacct gagacaagca gccagcagca gctcctgagc cccacgctgt 4980
cggatcgagg aggaagtcgg caagatgcag ccgacgcagg gaaaccccag aggaaatttg 5040
ggcagtggcg tctgccctca gccccaaaac caataagcca ttcagtgtcc tcagtcaact 5100
tacggtttgg aggaaggaca accatgaaat ctgtcgtgtg caaaatgaac cccatgactg 5160
acgcggcttc ctgcggttct gaagttaaga agtggtggac ccggcagctg actgtggaga 5220
gcgacgaaag tggggatgac cttctggata tttaggtgga tgtcaatgta gatgaatttc 5280
tagtggtgga aaccgttttc taataatgtc cttgattgtc cagtgagcaa tctgtaattg 5340
atctataact gaattccagc ttgtcacaag atgtttataa attgattttc atcctgccac 5400
agaaaggcat aagctgcatg tatgatgggt tactatcaat cattgctcaa aaaaattttt 5460
gtataatgac agtactgata atattagaaa tgataccgca agcaaatgta tatcacttaa 5520
aaatgtcata tattctgtct gcgtaaacta aggtatatat tcatatgtgc tctaatgcag 5580
tattatcacc gccccgcaaa agagtgctaa gcccaaagtg gctgatattt agggtacagg 5640
ggttatagct ttagttcaca tctttcccat ttccactaga aatatttctc gagagaattt 5700
attatttatg attgatctga aaaggtcagc actgaactta tgctaaaatg atagtagttt 5760
```

tacaaactac agattctgaa ttttaaaaag tatcttcttt ttctcgtgtt atatttttaa 5820
atatacacaa gacatttggt gaccagaaca agttgatttc tgtcctcagt tatgttaatg 5880
aaactgttgc ctccttctaa gaaaattgtg tgtgcaagca ccaggcaaag aaatggactc 5940
aggatgctta gcggtttgaa acaaacctgt agataaatca cttgagtgac atagttgcgc 6000
aaagatgtta agtttcttaa gaaacctttt aataactgag tttagcaaaa agaataaaac 6060
tatatagctc aatttattta aaaaaatctt tttgcatgtg tgatgttatc attggcttca 6120
tttcttaccc aaggtatgtc tgttttgcca taaatcagca gagtcatttc attctgggtg 6180
atcctgacac accattgcta tgttagattt gaaatgacat ctctgttaaa agaatcttct 6240
atggaaataa tggtgccctg caaaatcttc ctctgaactc acaggttagg gatcacacaa 6300
cttacttaat cgtttttgt ttttgttttt tttccttata tgtcaatggc ccatgtcctc 6360
cgggaaaatt agaaaagcaa aatgattaca aagtgctgtt agatttcttg tgctgggcca 6420
gccaagtaga agtggacttg acttggacct ttaactattt tattacagat tggacatttg 6480
ctgttcagat gtttttaac agagggatta tctcagaatc ctgtgacctc caggttgttt 6540
tataatctat ttttctctat ttaacattcc tcagatagat aggcaaatag gacattcctt 6600
ctgtgtcaca gaagtatcgt ggtagtggca gtctacagtt tatatgattc attgtaacta 6660
tgagataaag aacaaccagt catgtggcca aaaggattag atttgattgg atgttcactt 6720
ggagtttact ttttgtacat acaagataaa ataaatattg gatttgtaaa ataaaaaaaa 6780
aaaaaaaaa 6789

<210> 4
<211> 903
<212> DNA
<213> Homo sapiens

<400> 4
atgctaaatc tgcttgtagc cataattgtg gagaatttct ccttgtttta ttccactgag 60
gaggaccagc ttttaagtta caatgatctt cgccactttc aaatcatatg gaacatggtg 120
gatgataaaa gagagggggt gatccccacg ttccgcgtca agttcctgct gcggctactg 180
cgtgggaggc tggaggtgga cctggacaag gacaagctcc tgtttaagca catgtgctac 240
gaaatggaga ggctccacaa tggcggcgac gtcaccttcc atgatgtcct gagcatgctt 300
tcataccggt ccgtggacat ccggaagagc ttgcagctgg aggaactcct ggcgagggag 360
cagctggagt acaccataga ggaggaggtg gccaagcaga ccatccgcat gtggctcaag 420
aagtgcctga agcgcatcag agctaaacag cagcagtcgt gcagtatcat ccacagcctg 480
agagagagtc agcagcaaga gctgagccgg tttctgaacc cgcccagcat cgagaccacc 540
cagcccagtg aggacacgaa tgccaacagt caggacaaca gcatgcaacc tgagacaagc 600
agccagcagc agctcctgag ccccacgctg tcggatcgag gaggaagtcg gcaagatgca 660

```
gccgacgcag ggaaacccca gaggaaattt gggcagtggc gtctgccctc agccccaaaa 720
ccaataagcc attcagtgtc ctcagtcaac ttacggtttg gaggaaggac aaccatgaaa 780
tctgtcgtgt gcaaaatgaa ccccatgact gacgcggctt cctgcggttc tgaagttaag 840
aagtggtgga cccggcagct gactgtggag agcgacgaaa gtggggatga ccttctggat 900
att                                                               903
```

<210> 5

<211> 301

<212> PRT

<213> Homo sapiens

<400> 5

```
Met Leu Asn Leu Leu Val Ala Ile Ile Val Glu Asn Phe Ser Leu Phe
 1               5                  10                  15
Tyr Ser Thr Glu Glu Asp Gln Leu Leu Ser Tyr Asn Asp Leu Arg His
            20                  25                  30
Phe Gln Ile Ile Trp Asn Met Val Asp Asp Lys Arg Glu Gly Val Ile
        35                  40                  45
Pro Thr Phe Arg Val Lys Phe Leu Leu Arg Leu Leu Arg Gly Arg Leu
    50                  55                  60
Glu Val Asp Leu Asp Lys Asp Lys Leu Leu Phe Lys His Met Cys Tyr
65                  70                  75                  80
Glu Met Glu Arg Leu His Asn Gly Gly Asp Val Thr Phe His Asp Val
                85                  90                  95
Leu Ser Met Leu Ser Tyr Arg Ser Val Asp Ile Arg Lys Ser Leu Gln
            100                 105                 110
Leu Glu Glu Leu Leu Ala Arg Glu Gln Leu Glu Tyr Thr Ile Glu Glu
        115                 120                 125
Glu Val Ala Lys Gln Thr Ile Arg Met Trp Leu Lys Lys Cys Leu Lys
    130                 135                 140
Arg Ile Arg Ala Lys Gln Gln Gln Ser Cys Ser Ile Ile His Ser Leu
145                 150                 155                 160
Arg Glu Ser Gln Gln Gln Glu Leu Ser Arg Phe Leu Asn Pro Pro Ser
                165                 170                 175
Ile Glu Thr Thr Gln Pro Ser Glu Asp Thr Asn Ala Asn Ser Gln Asp
            180                 185                 190
```

Asn Ser Met Gln Pro Glu Thr Ser Ser Gln Gln Gln Leu Leu Ser Pro
195 200 205

Thr Leu Ser Asp Arg Gly Gly Ser Arg Gln Asp Ala Ala Asp Ala Gly
210 215 220

Lys Pro Gln Arg Lys Phe Gly Gln Trp Arg Leu Pro Ser Ala Pro Lys
225 230 235 240

Pro Ile Ser His Ser Val Ser Ser Val Asn Leu Arg Phe Gly Gly Arg
245 250 255

Thr Thr Met Lys Ser Val Val Cys Lys Met Asn Pro Met Thr Asp Ala
260 265 270

Ala Ser Cys Gly Ser Glu Val Lys Lys Trp Trp Thr Arg Gln Leu Thr
275 280 285

Val Glu Ser Asp Glu Ser Gly Asp Asp Leu Leu Asp Ile
290 295 300

<210> 6
<211> 2471
<212> DNA
<213> Homo sapiens

<400> 6
ctgttcattt tatgtcatca ttgcctacat catgctaaat ctgcttgtag ccataattgt 60
ggagaatttc tccttgtttt attccactga ggaggaccag cttttaagtt acaatgatct 120
tcgccacttt caaatcatat ggaacatggt ggatgataaa agagaggggg tgatccccac 180
gttccgcgtc aagttcctgc tgcggctact gcgtgggagg ctggaggtgg acctggacaa 240
ggacaagctc ctgtttaagc acatgtgcta cgaaatggag aggctccaca atggcggcga 300
cgtcaccttc catgatgtcc tgagcatgct ttcataccgg tccgtggaca tccggaagag 360
cttgcagctg gaggaactcc tggcgaggga gcagctggag tacaccatag aggaggaggt 420
ggccaagcag accatccgca tgtggctcaa gaagtgcctg aagcgcatca gagctaaaca 480
gcagcagtcg tgcagtatca tccacagcct gagagagagt cagcagcaag agctgagccg 540
gtttctgaac ccgcccagca tcgagaccac ccagcccagt gaggacacga atgccaacag 600
tcaggacaac agcatgcaac ctgagacaag cagccagcag cagctcctga gccccacgct 660
gtcggatcga ggaggaagtc ggcaagatgc agccgacgca gggaaacccc agaggaaatt 720
tgggcagtgg cgtctgccct cagccccaaa accaataagc cattcagtgt cctcagtcaa 780
cttacggttt ggaggaagga caaccatgaa atctgtcgtg tgcaaaatga accccatgac 840
tgacgcggct tcctgcggtt ctgaagttaa gaagtggtgg acccggcagc tgactgtgga 900

gagcgacgaa agtggggatg accttctgga tatttaggtg gatgtcaatg tagatgaatt 960
tctagtggtg gaaaccgttt tctaataatg tccttgattg tccagtgagc aatctgtaat 1020
tgatctataa ctgaattcca gcttgtcaca agatgtttat aaattgattt tcatcctgcc 1080
acagaaaggc ataagctgca tgtatgatgg gttactatca atcattgctc aaaaaaattt 1140
ttgtataatg acagtactga taatattaga aatgataccg caagcaaatg tatatcactt 1200
aaaaatgtca tatattctgt ctgcgtaaac taaggtatat attcatatgt gctctaatgc 1260
agtattatca ccgccccgca aaagagtgct aagcccaaag tggctgatat ttagggtaca 1320
ggggttatag ctttagttca catctttccc atttccacta gaaatatttc tcgagagaat 1380
ttattattta tgattgatct gaaaaggtca gcactgaact tatgctaaaa tgatagtagt 1440
tttacaaact acagattctg aattttaaaa agtatcttct ttttctcgtg ttatattttt 1500
aaatatacac aagacatttg gtgaccagaa caagttgatt tctgtcctca gttatgttaa 1560
tgaaactgtt gcctccttct aagaaaattg tgtgtgcaag caccaggcaa agaaatggac 1620
tcaggatgct tagcggtttg aaacaaacct gtagataaat cacttgagtg acatagttgc 1680
gcaaagatgt taagtttctt aagaaacctt ttaataactg agtttagcaa aaagaataaa 1740
actatatagc tcaatttatt taaaaaaatc tttttgcatg tgtgatgtta tcattggctt 1800
catttcttac ccaaggtatg tctgttttgc cataaatcag cagagtcatt tcattctggg 1860
tgatcctgac acaccattgc tatgttagat ttgaaatgac atctctgtta aaagaatctt 1920
ctatggaaat aatggtgccc tgcaaaatct tcctctgaac tcacaggtta gggatcacac 1980
aacttactta atcgtttttt gtttttgttt tttttcctta tatgtcaatg gcccatgtcc 2040
tccgggaaaa ttagaaaagc aaaatgatta caaagtgctg ttagatttct tgtgctgggc 2100
cagccaagta gaagtggact tgacttggac ctttaactat tttattacag attggacatt 2160
tgctgttcag atgtttttta acagagggat tatctcagaa tcctgtgacc tccaggttgt 2220
tttataatct atttttctct atttaacatt cctcagatag ataggcaaat aggacattcc 2280
ttctgtgtca cagaagtatc gtggtagtgg cagtctacag tttatatgat tcattgtaac 2340
tatgagataa agaacaacca gtcatgtggc caaaaggatt agatttgatt ggatgttcac 2400
ttggagttta ctttttgtac atacaagata aaataaatat tggatttgta aaataaaaaa 2460
aaaaaaaaaa a 2471